# EUROPÄISCHE PATENTSCHRIFT

(11) **EP 0 664 452 B1**
(45) Veröffentlichungstag und Bekanntmachung des Hinweises auf die Patenterteilung: **31.07.2002**
(21) Anmeldenummer: 95100632.9
(22) Anmeldetag: 18.01.1995
(51) Int. Cl.: G01N 33/543, G01N 33/547, C12Q 1/68, G01N 33/52, C07F 7/08, C07F 7/18

(54) **Biotinsilan-Verbindungen und diese Verbindungen enthaltende Bindematrix**
Biotin-silane compounds and binding matrix containing these compounds
Composés biotine-silane et matrice de liaison contenant ces composés

(30) Priorität: 19.01.1994 DE 4401450; 06.10.1994 DE 4435728
(43) Veröffentlichungstag der Anmeldung: 26.07.1995
(73) Patentinhaber: Roche Diagnostics GmbH, 68305 Mannheim (DE)
(72) Erfinder: Sluka, Dr. Peter, D-82362 Weilheim (DE); Batz, Dr. Hans-Georg, D-82327 Tutzing (DE)
(74) Vertreter: Weiss, Wolfgang, Dipl.-Chem. Dr.

(56) Entgegenhaltungen:
- EP-A- 0 325 404
- EP-A- 0 337 081
- WO-A-92/10757
- DE-A- 4 018 523
- US-A- 5 851 840
- TETRAHEDRON LETTERS, Bd. 32, Nr. 14, April 1991, OXFORD GB, Seiten 1715-1718, XP000579627 R. T. PON: "A long chain biotin phosphoramidite reagent for the automated synthesis of 5'-biotinylated oligonucleotides."
- BIOCHEMISTRY, Bd. 28, 3.Oktober 1989, EASTON, PA US, Seiten 8214-8221, XP000579671 R. BLANKENBURG ET AL.: "Interaction between biotin lipids and streptavidin in monolayers: formation of oriented two-dimensional protein domains induced by surface recognition."
- JOURNAL OF ORGANIC CHEMISTRY, Bd. 56, Nr. 24, 22.November 1991, EASTON US, Seiten 6850-6856, XP000579628 W. LIN & T. H. MORTON: "Two-step affinity chromatography. Model systems and an example using biotin-avidin binding and a fluoridolyzable linker."
- Silane coupling agents, Edwin P. Plueddermann, 1982, Plenum Press, New York, Kapitel 8.4, Seiten 214-217

## Beschreibung

Die vorliegende Erfindung betrifft eine Bindematrix, die ein Trägermaterial mit einer oxidischen Oberfläche und einen daran über Ankergruppen kovalent gebundenen Festphasen-Reaktanten, der mit mindestens einem freien Reaktionspartner bindefähig ist, enthält, wobei dieser Festphasen-Reaktant eine verdünnte Bindeschicht auf der Oberfläche des Trägermaterials bildet.

Bei molekularen Erkennungsreaktionen handelt es sich um die feste und spezifische Bindung zweier Moleküle, die ohne die Ausbildung einer kovalenten Atombindung eintritt. Für die praktische Handhabung sind insbesondere solche Reaktionen von Interesse, die an der Grenzfläche zwischen einem festen Trägermaterial und einer fluiden Umgebung ablaufen. Dazu wird die Oberfläche des festen Trägermaterials mit einer Fixierschicht belegt, die einen Festphasen-Reaktanten enthält. An dieser Fixierschicht laufen dann die eigentlichen Erkennungsreaktionen ab.

Ein Beispiel für eine derartige Erkennungsreaktion ist ein Festphasenimmunoassay, der über einen an der Festphase fixierten Antikörper durchgeführt wird. Dieser Antikörper muß bei diversen Waschprozeduren stabil an der Oberfläche der Festphase haften und dennoch eine genügend hohe immunologische Aktivität aufweisen. Üblicherweise werden als Festphasen für Immunoassays Kunststoffoberflächen, vorzugsweise aus Polystyrol, verwendet. Zwei Arten von Bindung sind entscheidend für die Brauchbarkeit der Festphase als Bindematrix für eine molekulare Erkennungsreaktion, nämlich zum einen eine möglichst hohe Bindung von spezifischen Antikörpern, die z.B. auf Polystyrol adsorbtiv aufgebracht werden und zum anderen eine möglichst geringe unspezifische Bindung von Bestandteilen einer Probeflüssigkeit, z.B. Blut- und Serumbestandteilen, wie Albumin und insbesondere Fibrinogen.

Die unspezifische Bindung von Proteinen an Oberflächen wird durch hydrophobe, polare oder Ladungstransfer-Wechselwirkungen sowie durch Wasserstoff-Brückenbindungen verursacht, wobei je nach Art des Proteins die eine oder die andere Bindungsart dominierend ist. Diese unspezifische Bindung von Proteinen stellt ein großes Problem bei diagnostischen Tests, z.B. Festphasenimmunoassays und auch bei medizinischen Implantaten dar. In der Vergangenheit wurden zur Lösung des Problems der unspezifischen Proteinbindung eine sehr große Zahl von Vorschlägen in der Literatur publiziert. In allen beschriebenen Verfahren erreicht man jedoch nur eine teilweise Beseitigung der unspezifischen Bindung bzw. eine Verschiebung von einer Bindungsart zur anderen (vgl. z.B. Baeyer et al., Trans. Am. Soc. Artif. Intern. Organs 26 (1980), 309; Gott et al., Federation Proceedings 30 (1977), 5, 1679; Wojciechowski et al., Colloids and Surfaces B: Biointerfaces 1 (1993), 107-117).

EP 0 325 404 A2 beschreibt ein Verfahren zur Immobilisierung von physiologischen Wirkstoffen an einen anorganischen Träger, der mit einem Aminoalkylalkoxysilan beschichtet ist. Die dichtgepackten Schichten gemäß Ep 0 325 405 weisen ebenfalls ein hohes Maß an unspezifischer Bindung und zudem nur eine relativ geringe Bindekapazität im Hinblick auf die gewünschte Bindung des Analyten auf.

Von Whitesides et al. (Science 252 (1991), 1164-1166) wurde gezeigt, daß auf Gold- oder Silberoberflächen durch Selbstassemblierung von Thiolen, die mit Oligoethylenoxideinheiten funktionalisiert sind, eine unspezifische Bindung von Fibrinogen auf Metalloberflächen vollständig unterdrückt werden kann.

Wie bereits erwähnt, spielt die unspezifische Bindung von Proteinen in der Diagnostik, speziell in Festphasenimmunoassays, eine große Rolle. Besonders vorteilhaft als Festphase, weil universell verwendbar, erwies sich für solche Anwendungen eine Fixierschicht aus Streptavidin, die durch Bindung mit Biotin bzw. biotinylierten Reaktanten für eine große Anzahl von Immuntests verwendet werden kann. Eine besonders gute Adsorption wurde durch Verwendung von an polymerisiertes Albumin gebundenem Streptavidin erreicht. Zur Unterdrückung der unspezifischen Bindung an diese Festphase kann ein oberflächenaktives Mittel zugesetzt werden (WO88/07683).

Bei neuen Testsystemen, wie z.B. optischen Biosensoren, sind jedoch Kunststoffoberflächen als Festphasen oftmals nicht geeignet. Für diese Anwendungen sind Trägermaterialien mit einer oxidischen Oberfläche, z.B. SiO₂ oder TiO₂, von besonderem Interesse. Für diese Festphasen sind ebenfalls verschiedene Methoden zur Immobilisierung von immunologisch aktiven Substanzen beschrieben. Da oxidische Oberflächen für eine direkte adsorptive Beschichtung normalerweise zu hydrophil sind, erfolgt die Beschichtung entweder adsorptiv nach Hydrophobisieren der Oberfläche (WO91/16425, Anal. Biochem. 145 (1985), 106-112), lichtinduziert (EP-A-0337081) oder kovalent über eine entsprechende Funktionalisierung der Oberfläche (Sensor 91, Kongress Band IV).

In der PCT-Anmeldung WO 92/10757 wird eine Bindematrix offenbart, die ein Trägermaterial und einen daran über Ankergruppen adsorbierten Festphasen-Reaktanden, der mit mindestens einem freien Reaktionspartner bindefähig ist, enthält, wobei der Festphasenreaktand eine verdünnte und im wesentlichen lateral homogene Bindeschicht auf der Oberfläche des Trägermaterials bildet. Als Festphasen werden metallische Oberflächen, z.B. Gold, und als Festphasenreaktanden Biotinthiole beschrieben. Die Verdünnung der Bindeschicht auf der Oberfläche kann durch Coadsorption eines Biotinthiols und eines nicht-biotinylierten Thiols erfolgen. Diese Bindematrix ist bei geeigneter Mischung von Biotinkomponente und Verdünner in der Lage, eine Streptavidin-Monoschicht mit einer Schichtdicke von 4 nm spezifisch auf der Oberfläche zu binden. Die unspezifische Bindung von Streptavidin wird durch geeignete Wahl des Verdünners vermieden.

Ein Nachteil der in WO 92/10757 beschriebenen Bindematrix besteht darin, daß eine adsorptive Beschichtung auf hydrophilen Oberflächen nur sehr schwierig durchführbar ist. Dort wird ein kontinuierliches Abbluten der Proteinschicht, insbesondere in Gegenwart von oberflächenaktiven Mitteln, beobachtet. Dies trifft teilweise auch auf hydrophobisierte Oberflächen zu.

Neben der Anwendung in Festphasenimmunoassays sind unspezifische Proteinbindungen auf oxidischen Oberflächen noch bei einer Reihe von weiteren Anwendungen relevant, insbesondere z.B. bei Oberflächen von Blutfiltern und Bauteilen aus Glas in medizinischen Geräten (z.B. Dialysegeräten), die direkt mit Blut oder Blutbestandteilen in Berührung kommen, bei Implantaten aller Art (z.B. Hüftgelenken, Herzklappen), die aus Titan bzw. Titanlegierungen oder Keramiken bestehen, sowie bei Kanülen und chirurgischen Instrumenten. Die unspezifische Anlagerung von Proteinen ist der Primärschritt zur Verkapselung der Implantate und begrenzt deren Verweildauer im Körper auf ca. 10 Jahre (Ullmann, Enzyclopädie der chemischen Technik, Band 18, Seite 169 ff.).

Weiterhin ist die unspezifische Bindung an oxidischen Oberflächen auch bei Chromatographiematerialien, Trägermaterialien für Peptid- oder Oligonukleotidsynthesen sowie bei Laborglasmaterialien, die direkt mit Blut in Berührung kommen (Zentrifugengläser etc.), relevant. Auch außerhalb der Medizin besteht das Problem der unerwünschten Adsorption an hydrophile Oberflächen, z.B. bei Autoscheiben, Geschirr etc..

Alle bisher beschriebenen Verfahren zur Verringerung bzw. Unterdrückung der Adsorption an oxidische Oberflächen weisen - wie oben ausgeführt - verschiedene Nachteile auf. Insbesondere Immuntests auf oxidischen Oberflächen zeigen verstärkt das Problem eines Abblutens von adsorbtiv gebundenen Antikörpern und bei kovalenter Bindung eine unkontrollierte Stöchiometrie und daneben die Problematik der unspezifischen Proteinadsorption.

Das der vorliegenden Erfindung zugrundeliegende technische Problem bestand somit darin, oxidische Oberflächen auf derartige Weise zu modifizieren, daß das Problem einer unspezifischen Adsorption von Proteinen beseitigt oder doch zumindest erheblich verringert wird.

Ein Aspekt der vorliegenden Erfindung betrifft die Bereitstellung einer Bindematrix auf Basis eines Trägermaterials mit einer oxidischen Oberfläche, bei der das Abbluten von Festphasen-Reaktanden verhindert und die unspezifische Adsorption von Proteinen verringert wird.

Völlig überraschend wurde festgestellt, daß bei einer Funktionalisierung von oxidischen Oberflächen mit bestimmten reaktiven Silanverbindungen die unspezifische Bindung von Proteinen, insbesondere die unspezifische Bindung von Fibrinogen, stark vermindert oder vollständig beseitigt werden kann. Hierzu wird die oxidische Oberfläche mit Verbindungen umgesetzt, die oberflächenreaktive Silan-Endgruppen als Ankergruppen zur Reaktion mit der Festphase sowie eine Alkoxy(oligoalkylenoxid)gruppe oder eine Festphasen-Reaktandengruppe aufweisen, wobei die Ankergruppe mit der Festphasen-Reaktandengruppe bzw. mit der Alkoxy(oligoalkylenoxid)gruppe über ein Spacermolekül verknüpft ist.

Weiterhin wurde festgestellt, daß auf diese Weise hergestellte Bindematrix reproduzierbar und steuerbar mit einer definierten Menge an Festphasenreaktanden belegt werden kann, wobei die Festphasenreaktanden ihrerseits wieder die Grundlage für eine reproduzierbare Belegung mit einer oder mehreren Schichten von freien Reaktionspartnern dienen können. Auf diese Weise wird ein Analysenelement erhalten, das ebenso wie die Bindematrix zum Nachweis von freien Analyten dienen kann.

Überdies besitzt die Bindematrix bzw. das Analysenelement nicht nur eine konstante Homogenität über größere Flächenbereiche (Makrohomogenität), sondern auch innerhalb sehr kleiner Flächenbereiche (Mikrohomogenität). Somit können auf einfache Weise kompartimentierte Bindematrices bzw. Analysenelemente hergestellt werden, die sich hervorragend zum multiplen Analytennachweis eignen.

Ein erster Aspekt der vorliegenden Erfindung betrifft somit eine Bindematrix, enthaltend ein Ankermaterial mit einer oxidischen Oberfläche und einen daran über Ankergruppen kovalent gebundenen Festphasen-Reaktanten, der mit mindestens einem freien Reaktionspartner bindefähig ist, welche dadurch gekennzeichnet ist, daß der Festphasen-Reaktant eine verdünnte und im wesentlichen lateral homogene Bindeschicht auf der Oberfläche des Trägermaterials bildet und daß die Ankergruppen Silangruppen sind, die mit dem Festphasen-Reaktanten über ein Spacermolekül verknüpft sind und dass die Bindematrix neben den mit Festphasen-Reaktanten verknüpften Spacermolekülen noch Verdünnermoleküle enthält, die mit Ankergruppen kovalent an die Festphase gebunden sind.

In einer bevorzugten Ausführungsform besteht die verdünnte Monoschicht aus einer Molekülsorte, wobei die Oberfläche nicht vollständig belegt ist. Der Belegungsgrad mit dem Festphasenreaktanten, der ein Maß für die "Verdünnung" ist, kann ausgedrückt werden als Quotient aus gefundener Dicke der Monoschicht dividiert durch die theoretische Schichtdicke bei dichter Packung.

Der Belegungsgrad der Monoschichten mit dem Festphasen-Reaktanten ist kleiner als 100 %, vorzugsweise 0,1 bis 90 %, besonders bevorzugt 0,5 bis 70 %, am meisten bevorzugt 1 bis 40 %.

Durch den relativ großen Abstand der einzelnen Moleküle des Festphasen-Reaktanten auf der Oberfläche des Trägermaterials enthält die erfindungsgemäße Bindematrix eine aufgelockerte Schicht im Gegensatz zur dichtgepackten Schicht des Standes der Technik. Die verdünnte Monoschicht an der Oberfläche der erfindungsgemäßen Bindematrix ermöglicht eine schnellere Bindung des freien Reaktionspartners aus einer fluiden Phase und zeichnet sich überraschenderweise durch eine höhere Bindekapazität aus.

Das Trägermaterial der erfindungsgemäßen Bindematrix weist eine oxidische Oberfläche auf. Beispiele für oxidische Oberflächen sind Oxide von Metallen oder Halbmetallen (z.B. B oder Si) in Form von reinen Oxiden oder Mischungen davon (z.B. Glas). Spezifische Beispiele sind SiO₂, TiO₂, Al₂O₃, Fe₂O₃, Ag₂O, Cr₂O₃, Ta₂O₅ und Mischungen davon, wobei SiO₂, TiO₂ und Mischungen davon besonders bevorzugt sind.

Die kovalente Bindung des Festphasenreaktanden an die Oberfläche des Trägermaterials wird durch Ankergruppen vermittelt. Die Ankergruppen sind oberflächenreaktive Silan-Endgruppen, d.h. Gruppen, in den ein oder mehrere Siliziumatome mit Substituenten vorliegen, die eine Reaktion mit einer oxidischen Oberfläche eingehen können. Beispiele für derartige Substituenten sind Alkoxy-, insbesondere C₁-C₄-Alkoxygruppen und Halogen-, insbesondere Chloratome. Eine besonders bevorzugte reaktive Endgruppe ist eine Silangruppe mit der allgemeinen Formel (I) :

R¹R²R³Si- (I)

worin R¹, R² und R³ gleich oder verschieden sein können und Substituenten des Siliziumatoms darstellen, unter der Vorraussetzung, daß mindestens einer der Substituenten R¹, R² und R³ eine Reaktion mit einer oxidischen Oberfläche eingehen kann. Spezifische Beispiele für Silan-Engruppen der allgemeinem Formel (I) sind Monoalkoxydialkylsilan-, Dialkoxymonoalkylsilan-, Trialkoxysilan- oder Trihalogensilangruppen, wobei die Alkyl- und Alkoxyreste vorzugsweise, 1 bis 4 C-Atome, besonders bevorzugt 1 oder 2 C-Atome, und am meisten bevorzugt 1 C-Atom, aufweisen. Bevorzugte spezifische Beispiele sind daher Monomethoxydimethylsilan-, Dimethoxymonomethylsilan-, Trimethoxysilan- sowie Chlorsilan-Endgruppen. Vorzugsweise ist die Silangruppe (I) eine trifunktionelle Gruppe, d.h. alle 3 Substituenten können eine Reaktion mit der oxidischen Oberfläche eingehen, z.B. Trimethoxysilan.

Andererseits kann die Ankergruppe auch durch Reaktion der oxidischen Trägermaterialoberfläche mit einer oberflächenreaktiven Silangruppe der allgemeinen Formel (II) gebildet werden: 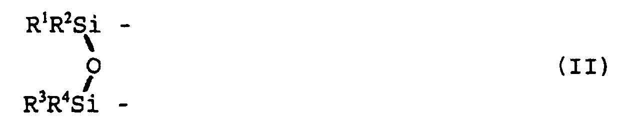 worin R¹, R², R³ und R⁴ entsprechend der Definition in Formel I sind, unter der Vorraussetzung, daß mindestens einer der Substituenten R¹ und R² oder R³ und R⁴ eine kovalente Bindung mit einer oxidischen Oberfläche eingehen kann. Spezifische Beispiel für reaktive Silan-Endgruppen der Formel (II) sind Tetramethyldisilanoxy- oder Tetramethoxydisilanoxygruppen. Vorzugsweise ist die Silangruppe (II) eine tetrafunktionelle Gruppe, d.h. alle 4 Substituenten können eine Reaktion mit der oxidischen Oberfläche eingehen, z.B. Tetramethoxydisilanoxy.

Die Ankergruppe ist mit dem Festphasenreaktanden über ein Spacermolekül, vorzugsweise über ein flexibles lineares Spacermolekül mit einer Kettenlänge von 2 bis 50, insbesondere 4 bis 40 Atomen verknüpft. Besonders bevorzugt umfaßt das Spacermolekül eine gegebenenfalls substituierte oder/und Heteroatome enthaltende Alkylengruppe. Als Substituenten kommen zweckmäßigerweise nur solche Atome oder Gruppen in Frage, die nicht mit der oxidischen Oberfläche eine kovalente Bindung eingehen können, z.B. Halogenatome oder zweifach gebundener Sauerstoff. Beispiele für Heteroatome sind insbesondere N, O und S, wobei N und O bevorzugt sind.

Auf der einen Seite des Spacermoleküls befindet sich die Ankergruppe, die kovalent an die Oberfläche des Trägermaterials gebunden ist. Auf seiner anderen Seite, d.h. vom Trägermaterial wegstehend, enthält das Spacermolekül eine oder mehrere Verknüpfungsgruppierungen, über die der Festphasen-Reaktant oder eine Komponente davon mit dem Spacermolekül verknüpft ist. Bei diesen Verknüpfungsgruppierungen kann es sich z.B. um eine Amino- oder Hydroxylfunktion handeln, die z.B. mit einer Carboxylfunktion des Festphasen-Reaktanden unter Bildung einer Ester- oder Amidgruppe verknüpft ist. Das Spacermolekül kann jedoch auch als Verknüpfungsgruppe eine Carboxylfunktion enthalten, die dann wiederum mit einer reaktiven Amino- oder Hydroxylfunktion des Festphasenreaktanden verknüpft ist.

Eine bevorzugte Möglichkeit zur Herstellung einer erfindungsgemäßen Bindematrix ist der Einbau einer hydrophilen Linkergruppe zwischen dem Spacermolekül und der Festphasen-Reaktanten. Dieser Linker ist insbesondere ein geradkettiges Molekül mit einer Kettenlänge von 4 bis 15 Atomen. Bevorzugt ist dabei eine Linkergruppe, die eine oder mehrere hydrophile Ethylenoxideinheiten, vorzugsweise zwischen 1 und 5 enthält. Besonders bevorzugt wird die hydrophile Linkergruppe durch ein Amin- oder Hydroxyl-terminiertes Polyethylenoxid gebildet. Als besonders geeigneter Linker hat sich 1,8-Diamino-3,6-dioxaoctan (DADOO) erwiesen.

Zwischen dem hydrophilen Linker und dem Festphasen-Reaktanten kann gegebenenfalls noch ein weiteres Spacermolekül eingebaut werden, das wie oben definiert ist.

Es soll klargestellt werden, daß für die Herstellung einer erfindungsgemäßen Bindematrix mit einer verdünnten Monoschicht des Festphasen-Reaktanten mehrere Möglichkeiten bestehen. Im folgenden werden einige dieser Möglichkeiten aufgeführt.

Eine erste Möglichkeit zur Herstellung der erfindungsgemäßen verdünnten Bindematrix besteht in der Wahl geeigneter Beschichtungsbedingungen. So kann durch Variation von Parametern, wie der Konzentration des Festphasenreaktanden, der mit der oxidischen Oberfläche in Kontakt gebracht wird, den Reaktionsbedingungen, wie etwa Temperatur, Reaktionsdauer oder der Reaktivität der Ankergruppen des Festphasen-Reaktanten, eine geeignete Verdünnung der Bindeschicht an der Oberfläche des Trägermaterials erreichen. Bezüglich dieser Parameter ist festzustellen, daß die Belegungsdichte des Festphasen-Reaktanten auf der Oberfläche des Trägermaterials bei Erhöhung der Konzentration des Festphasen-Reaktanten, der Reaktionstemperatur, der Reaktionsdauer und der Reaktivität der Ankergruppen ansteigt, während umgekehrt bei Erniedrigung dieser Parameter eine Verringerung der Belegungsdichte erreicht werden kann. Die zur Herstellung einer verdünnten Bindematrix geeigneten Reaktionsbedingungen sind im folgenden ausführlicher beschrieben.

Erfindungsgemäß wird die Herstellung einer verdünnten Bindeschicht des Festphasen-Reaktanten durch gleichzeitige Bindung von Molekülen des Festphasen-Reaktanten und von Verdünnungsmolekülen an die Oberfläche des Trägermaterials erreicht.

In dieser Ausführungsform enthält die erfindungsgemäße Bindematrix zusätzlich noch Spacermoleküle, die zwar mit einer Ankergruppe versehen sind, aber an die kein Festphasen-Reaktant gebunden ist. Derartige Verbindungen werden im weiteren auch als Verdünnungsmoleküle bezeichnet. Wenn man z.B. biotinylierte und nicht biotinylierte Spacermoleküle im Verhältnis von 1:10 bis 1:2 einsetzt, so erhält man eine verdünnte Biotin-Monoschicht, die den freien Reaktionspartner mit hoher Geschwindigkeit und großer Kapazität binden kann.

Geeignete Verdünnungsmoleküle enthalten eine Ankergruppe und einen Spacerbestandteil sowie ggf. ein Linkermolekül, wobei sich die Kettenlänge des Spacermoleküls des Verdünnungsmoleküls um nicht mehr als 1 - 5, bevorzugt nicht mehr als 1 - 2 Atome von der Kettenlänge des Spacermoleküls unterscheidet, das an den Festphasen-Reaktanten gebunden vorliegt. Es hat sich weiterhin als zweckmäßig erwiesen, daß die Mindestkettenlänge des Verdünnungsmoleküls 6 Atome (ohne Ankergruppe und hydrophile Linkergruppe) beträgt.

Anstelle des Festphasen-Reaktanten befindet sich vorzugsweise an dem von der Ankergruppe entfernten Ende des Verdünnungsmoleküls eine funktionelle Gruppe, wie z. B. eine Hydroxylgruppe, eine Carbonsäuregruppe, eine Carbonsäureethylester- oder Methylestergruppe, eine Carbonsäureamidgruppe, eine mit 1 oder 2 Methyl- oder Ethylgruppen substitutierte Carbonsäureamidgruppe, eine Sulfonsäuregruppe, eine Sulfonamidgruppe oder eine Alkoxyoligoalkylenoxidgruppe, insbesondere eine C₁-C₄-Alkoxy(C₂-C₄-alkylenoxid)gruppe mit 1 - 10 Alkylenoxideinheiten. Besonders bevorzugt sind Methoxyoligoalkylenoxidgruppen, wobei eine Alkylenoxidgruppe 2 bis 4 C-Atome, am meisten bevorzugt 2 C-Atome aufweisen kann. Die Anzahl der Alkylenoxidgruppen beträgt vorzugsweise 1 bis 10. Am meisten bevorzugt sind Methoxymonoethylenoxid-, Methoxydiethylenoxid- und Methoxytriethylenoxid- und Methoxytetraethylenoxidgruppen. Vorzugsweise enthält ein bevorzugtes Verdünnungsmolekül somit auf der einen Seite des Spacerbestandteils eine mit dem Trägermaterial reaktive Ankergruppe und auf der anderen Seite eine hydrophile Endgruppe.

Weiterhin können ein Spacer mit Festphasen-Reaktant und ein Spacer ohne Festphasen-Reaktant über eine kovalente Bindung verknüpft werden. Diese Verknüpfung erfolgt vorzugsweise über eine Si-O-Si-Brücke.

In solchen gemischten Monoschichten, die aus Verdünnungsmolekülen (Spacermolekülen ohne Festphasen-Reaktant) und aus Spacermolekülen mit Festphasen-Reaktant bestehen, beträgt der Anteil der Spacermoleküle mit Festphasen-Reaktant zweckmäßig 0,1 - 90 mol-%, vorzugsweise 0,5 - 50 mol-% und besonders bevorzugt 1 - 40 mol-%.

In allen bisher genannten Bindefilmen besteht der Festphasen-Reaktant aus einer Komponente. Dabei handelt es sich vorzugsweise um Biotin oder Biotin-analoge Moleküle wie Desthiobiotin, Iminobiotin oder HABA (4-Hydroxy-phenyl-azo-benzoesäure), die ebenfalls mit Streptavidin reagieren.

Weitere Beispiele für geeignete Festphasen-Reaktanten sind jedoch auch mit einem Antikörper bindefähige Antigene oder Haptene. In diesem Fall ist der Festphasen-Reaktant vorzugsweise ein Hapten mit einem Molekulargewicht von 100 bis 1200. Geeignet sind beispielsweise Steroide (wie z.B. Digoxin, Digoxigenin, Cortisol, Oestriol, Oestradiol, Theophyllin, Diphenylhydantoin, Testosterol, Gallensäuren, Progesteron und Aldosteron); kurzkettige Peptide (wie z.B. Argipressin, Oxytocin und Bradykinin); Fluorescein und seine Derivate; T3, T4, Aflatoxin, Atrazin, Pflanzenhormone wie z.B. Gibberilline; und Alkaloide (wie z.B. Reserpin und Ajmalicin). Weitere Beispiele für geeignete Festphasenreaktanden sind auch Oligo- und Polynukleotide, die mit freien Nukleinsäuren komplementärer Sequenz binden können.

Besonders bevorzugt werden als Hapten Biotin und Biotinderivate, Digoxin, Digoxigenin, Fluorescein und Derivate sowie Theophyllin eingesetzt.

Andererseits kann der Festphasen-Reaktant auch aus mehreren Komponenten bestehen. Darunter ist insbesondere zu verstehen, daß eine innere Komponente des Festphasen-Reaktanden kovalent mit einem Spacermolekül verknüpft ist und nicht kovalent an die äußere Komponente des Festphasen-Reaktanden gebunden ist. Dabei ist dann die äußere Komponente des Festphasen-Reaktanden zur Bindung eines freien Reaktionspartners in der Lage. Beispielsweise kann es sich bei der inneren Komponente um Biotin und bei der äußeren Komponente um Streptavidin handeln. Eine solche Bindematrix ist wiederum in der Lage, biotinylierte Reaktionspartner aus einer Lösung zu binden, da Streptavidin vier Bindestellen für Biotin besitzt, von denen mindestens zwei noch frei sind.

Eine Bindeschicht, die einen aus zwei Komponenten bestehenden Festphasen-Reaktanden enthält, ist dann eine erfindungsgemäße Bindematrix, wenn die äußere, d.h. die mit einem freien Reaktionspartner bindefähige Komponente des Festphasen-Reaktanden (d.h. im speziellen Fall Streptavidin) eine verdünnte Schicht an der Oberfläche der Bindematrix bildet. Vorzugsweise bildet die innere Komponente des Festphasen-Reaktanden eine unverdünnte Schicht an der Oberfläche der Bindematrix, an die sich die äußere Komponente des Festphasen-Reaktanden unter Bildung einer verdünnten Schicht anlagern kann.

So bindet eine dichtgepackte Biotinmonoschicht mit 100 % Belegung (die selbst keine erfindungsgemäße Bindematrix darstellt) Streptavidin mit einer geringeren Belegungsdichte. Diese verdünnte Streptavidin-Schicht stellt dann wiederum eine erfindungsgemäße Bindematrix dar, die einen freien Reaktionspartner, z.B. einen biotinylierten Antikörper zu einem dichtgepackten Film binden kann. Eine verdünnte Biotinmonoschicht, die beispielsweise durch Verwendung eines Festphasen-Reaktanden mit Spacer und Linker hergestellt wurde und die selbst eine erfindungsgemäße Bindematrix für die Bindung von freiem Streptavidin darstellt, kann Streptavidin zu einem dichtgepackten Film mit 100 % Belegung binden. Die dabei resultierende, dichtgepackte Streptavidinschicht stellt jedoch wiederum keine erfindungsgemäße Bindematrix dar (weil nicht flexibel) und kann einen Reaktionspartner (z.B. einen biotinylierten Antikörper) nur zu einem ca. 10 % belegten Film binden.

Dieses erfindungsgemäße Prinzip des verdünnten bindefähigen Festphasen-Reaktanden läßt sich von der Biotin-Streptavidin-Bindung auch auf andere Bindepaare, also z.B. Antikörper-Antigen etc. erweitern.

Der Belegungsgrad des Festphasen-Reaktanden an der Oberfläche der Bindematrix ist durch eine Dickenmessung der Bindeschicht bestimmbar. Dabei nimmt die gemessene Schichtdicke mit dem Belegungsgrad der Bindeschicht ab.

Die Vorteile der erfindungsgemäßen Bindematrix kommen überraschenderweise besonders gut in kleinen Flächenbereichen, beispielsweise in Flächen ≤ 0,5 cm² und insbesondere ≤ 0,1 cm² zur Geltung. Damit kann eine Bindematrix hergestellt werden, die eine Vielzahl von räumlich getrennten Bereichen bzw.

Kompartimenten aufweist, auf der mit ortsauflösenden Detektionsmethoden die Bindung von freien Reaktionspartnern an den Festphasenreaktanden sowohl qualitativ als auch quantitativ getrennt voneinander nachgewiesen werden kann.

Noch ein weiterer Gegenstand der vorliegenden Erfindung ist daher eine kompartimentierte Bindematrix, welche dadurch gekennzeichnet ist, daß auf einem Trägermaterial eine Vielzahl von räumlich getrennten bzw. diskreten Bereichen angeordnet ist, die jeweils eine erfindungsgemäße Bindematrix umfassen.

Die räumlich getrennten Bereiche bzw. Spots sind auf der gemeinsamen Trägermaterialoberfläche vorzugsweise in einem regelmäßigen Muster nebeneinander aufgebracht. Vorteilhaft sind 10-100 Spots pro cm² Oberfläche des Trägermaterials und besonders vorteilhaft sind 20-50 Spots. Der Durchmesser der Spots beträgt vorzugsweise 0,1 - 1 mm. Der Abstand zwischen den äußeren Begrenzungen der einzelnen Spots beträgt vorzugsweise 0,1 - 1 mm, um ein getrenntes Auftragen von Reagenzien zu erleichtern.

Vorzugsweise werden die räumlich getrennten, jeweils eine Bindematrix umfassenden Flächenbereiche auf ein Trägermaterial mit nicht-oxidischer Oberfläche aufgebracht. Vorzugsweise werden die Bereiche bzw. Spots in einem regelmäßigen Muster aufgebracht. Um das gewünschte Muster von Oxid-Spots auf der Trägermaterialoberfläche zu erhalten, kann beim Aufbringen der Spots eine Maske auf das Trägermaterial aufgelegt werden, in der das gewünschte Muster freigelassen ist. Die Maske kann anschließend mit oxidischem Material bedampft oder besputtert werden, wobei auf der nicht-oxidischen Trägermaterialoberfläche ein Muster mit Oxid-Spots entsteht.

Oxid-Spots können auch so hergestellt werden, daß eine oxidische Oberfläche mit nicht-oxidischem Material so bedeckt wird, daß das nicht-oxidische Material die Zwischenflächen zwischen einzelnen Spots des oxidischen Materials bildet.

Anschließend werden die Oxid-Spots auf der Trägeroberfläche mit der verdünnten erfindungsgemäßen Bindematrix belegt. In einer Ausführungsform der vorliegenden Erfindung enthalten die räumlich getrennten Oxid-Spots Bindematrices mit jeweils gleichen Festphasenreaktanden, wobei der Verdünnungsgrad des Festphasenreaktanden auf den einzelnen Spots gegebenenfalls variiert werden kann. Andererseits können die einzelnen räumlich getrennten Oxid-Spots auch Bindematrices mit unterschiedlichen Festphasenreaktanden umfassen.

Ein weiterer Aspekt der vorliegenden Erfindung ist ein Analysenelement, das eine verdünnte Bindematrix enthält, wobei der Festphasenreaktand mit einer oder mehreren zusätzlichen Schichten von Reaktionspartnern belegt ist. Überraschenderweise wurde nämlich festgestellt, daß ein erster freier Reaktionspartner gut reproduzierbar und homogen an den Festphasenreaktanden der erfindungsgemäßen Bindematrix binden kann. Über den Verdünnungsgrad des Festphasenreaktanden kann überraschenderweise sogar die Menge des Reaktionspartners über weite Bereiche variiert werden.

Nach Bindung einer definierten Menge des ersten freien Reaktionspartners an die Bindematrix kann das resultierende Analysenelement seinerseits noch weitere spezifische Reaktionspartner in einer weiteren Schicht sehr gut und reproduzierbar binden.

So kann beispielsweise eine erfindungsgemäße Bindematrix, die Biotin als Festphasenreaktanden enthält, mit Streptavidin reproduzierbar beschichtet werden und diese Streptavidinschicht kann ihrerseits wieder sehr gut reproduzierbar mit einer Schicht eines biotynilierten freien Reaktionspartners belegt werden. Der an den Festphasenreaktanden bindefähige Reaktionspartner ist vorzugsweise ein spezifischer bioaffiner Bindungspartner, der zum Nachweis von freien, sich in einer Probelösung befindlichen Analyten verwendet werden kann.

Beispiele für geeignete bindefähige Reaktionspartner sind z.B. Antikörper, Antigene, Haptene oder Nukleinsäuren.

In einer Ausführungsform kann eine kompartimentierte Bindematrix, die eine Vielzahl von räumlich getrennten Spots mit jeweils verdünnten Bindeschichten eines Festphasenreaktanden enthält, direkt zur reproduzierbaren Aufgabe von einer oder mehreren weiteren Schichten gleicher oder verschiedener Reaktionspartner dienen.

In einer anderen Ausführungsform werden die verdünnten Bindeschichten der einzelnen Spots jeweils durch eine weitere Bindeschicht belegt. Da diese Belegung erfindungsgemäß auch auf sehr kleiner Fläche reproduzierbar ist, können diese Bindeschichten sehr gut für die reproduzierbare Belegung einer einstellbaren Menge einer dritten Bindeschicht mit Reaktionspartnern dienen, die innerhalb des Analyseelements gleich oder von Spot zu Spot verschieden sein können.

Bei beiden Ausführungsformen kann jeder Spot des Analysenelements separat mit einer definierten Menge an Reaktionspartnern belegt werden, ohne daß unspezifische Bindungen diese Belegung stören. Selbst bei sehr kleinen Abmessungen der Spots kann eine homogene Belegung erreicht werden. Dadurch ist die Herstellung von Analysenelementen mit einer Vielzahl von Spots, die eine konstante und homogene Belegung eines Reaktionspartners aufweisen, auf sehr kleinem Raum möglich. Zusätzlich läßt sich die Belegung verschiedener Spots mit dem Reaktionspartner auch über den Verdünnungsgrad verschiedener Bindeschichten einfach und reproduzierbar steuern, so daß jeder Spot individuell bezüglich des nachzuweisenden Analyten und bezüglich der hierfür erforderlichen Sensitivität eingestellt werden kann.

Eine bevorzugte Ausführungsform der Erfindung ist daher ein multiples Analysenelement auf Basis einer kompartimentierten Bindematrix, das eine parallele qualitative oder/und quantitative Bestimmung einer Vielzahl von Analyten in einer Probe oder eines Analyten in verschiedenen Proben auf engem Raum erlaubt, wobei nur sehr geringe Reagenzmengen benötigt werden, so daß diese Bestimmungen nur sehr wenig durch unspezifische Bindungen von Blut- und Serumbestandteilen, wie z.B. Fibrinogen, gestört werden. Gegenüber konventionellen "Multiparameter"-Analysenelementen wie z.B. Mikrotiterplatten kann dabei eine wesentlich höhere Zahl von Analysenbereichen auf einer gegebenen Fläche angeordnet werden. So können bis zu 100 Bindematrix-Spots pro cm² problemlos angeordnet werden.

Das erfindungsgemäße kompartimentierte Analysenelement kann auf einer Bindematrix mit gleichen oder unterschiedlichen Festphasenreaktanden basieren. Eine bevorzugte Ausführungsform ist ein kompartimentiertes Analysenelement auf Basis einer Bindematrix, die auf jedem Spot den gleichen Festphasenreaktanden enthält. Jeder Spot kann seinerseits zur räumlich begrenzten und reproduzierbaren homogenen Bindung von gegebenenfalls unterschiedlichen freien Reaktionspartnern dienen.

Ein weiterer Gegenstand der vorliegenden Erfindung ist ein Analysenelement zur Bestimmung unterschiedlicher Analyten in einer Probe, umfassend eine kompartimentierte Bindematrix, worin die einzelnen räumlich getrennten Bindematrix-Bereiche mit jeweils unterschiedlichen, an den Festphasenreaktanden bindefähigen Reaktionspartnern belegt sind. Noch ein weiterer Gegenstand der vorliegenden Erfindung ist ein Analysenelement zur Bestimmung gleicher Analyten in verschiedenen Proben, umfassend eine kompartimentierte Bindematrix, worin die einzelnen räumlich getrennten Bindematrix-Bereiche mit jeweils gleichen, an die Festphasenreaktanden bindefähigen Reaktionspartnern belegt sind.

Die erfindungsgemäßen Multianalyt-Analyseelemente umfassen vorzugsweise ein flächenförmiges Trägermaterial, dessen Oberfläche mit einem regelmäßigen Muster räumlich getrennter Bereiche bedeckt ist, welche jeweils eine Bindematrix enthalten, die mit einer oder mehreren Schichten von zusätzlichen Reaktionspartnern belegt ist.

Weiterhin ein Gegenstand der vorliegenden Erfindung ist ein Verfahren zur Herstellung einer erfindungsgemäßen Bindematrix. Aufgrund der unterschiedlichen Natur der erfindungsgemäßen Bindematrizes sind auch die Herstellungsverfahren im Detail unterschiedlich. Mehrere bevorzugte Varianten dieser Herstellungsverfahren sind in den Ausführungsbeispielen dargestellt. Allgemein beinhaltet das erfindungsgemäße Verfahren die Inkubation des Trägermaterials mit einer Reaktionslösung, in der die Moleküle vorliegen, welche die Bindeschicht der erfindungsgemäßen Bindematrix bilden. Diese Moleküle enthalten an gegenüberliegenden Seiten die Ankergruppe und den Festphasen-Reaktanden (wobei in einigen Ausführungsformen der vorliegenden Erfindung nicht alle Moleküle der Bindeschicht mit einem Festphasen-Reaktanden verknüpft sein müssen). Vorzugsweise ist Festphasen-Reaktant über ein Spacermolekül mit der Ankergruppe verknüpft. Die Bindung der Ankergruppen an das Trägermaterial aus der Lösung unter Bildung der erfindungsgemäßen Bindematrix ist ein spontaner Prozeß.

Die Beschichtung von oxidischen Oberflächen mit Silanen erfolgt dadurch, daß eine Lösung der Silanverbindung bzw. des Gemisches von Silanverbindungen mit einer zuvor gereinigten oxidischen Oberfläche in Kontakt gebracht werden. Die Reinigung der oxidischen Oberfläche erfolgt vorzugsweise durch Kochen in einer starken anorganischen Säure, z.B. HNO₃, H₂SO₄, HCl und besonders bevorzugt Königswasser (H₂SO₄/HCl). Anschließend wird die oxidische Oberfläche zur Entfernung der Säure gewaschen und getrocknet. Dann wird die auf diese Weise gereinigte Oberfläche mit der Lösung der Silanverbindung in Kontakt gebracht. Als Lösungsmittel geeignet sind wasserfreie organische Lösungsmittel, wie etwa Methanol, Chloroform oder Mischungen davon. Weiterhin ist es bevorzugt, daß die Bindeschicht unter Schutzgas, z.B. N₂, aufgebracht wird.

Bei einer unvollständigen Silanisierung der oxidischen Oberfläche zur Erzeugung einer verdünnten Bindeschicht des Festphasenreaktanden ohne Verwendung eines Silanverdünners beträgt die Konzentration der Festphasenreaktand-Silanverbindung vorzugsweise 10⁻⁴ bis 10⁻² Gew.-% in einem geeigneten Lösungsmittel, besonders bevorzugt 5 x 10⁻³ Gew.-% bis 5 x 10⁻² Gew.-%. Die Reaktionsdauer beträgt vorzugsweise 10 bis 60 Minuten, besonders bevorzugt 20 bis 40 Minuten, und die Temperatur der Reaktion beträgt vorzugsweise 10 bis 50°C, besonders bevorzugt Raumtemperatur.

Wenn die oxidische Oberfläche mit einer Kombination aus Biotinsilan und Silanverdünner beschichtet werden soll, beträgt die Konzentration der Silanverbindungen im Lösungsmittel vorzugsweise 0,1 bis 5 Gew.-%, besonders bevorzugt 0,5 bis 2 Gew.-%, wobei die einzelnen Verbindungen in den jeweils gewünschten Anteilen eingesetzt werden. Die Reaktionszeit beträgt vorzugsweise 1 bis 8 Stunden, besonders bevorzugt 3 bis 5 Stunden. Die Temperatur beträgt vorzugsweise 10 bis 50°C, besonders bevorzugt Raumtemperatur.

Nach Durchführung der Reaktion werden die Oberflächen anschließend gespült, vorzugsweise mit dem zum Aufbringen des Silans verwendeten Lösungsmittels und für eine Dauer von 20 Minuten bis 8 Stunden bei erhöhter Temperatur, z.B. 60 bis 140°C in einem Ofen, gegebenenfalls unter Vakuum, konditioniert.

Gegebenenfalls kann in einem zweiten Schritt durch Inkubation mit einer zweiten Reaktionslösung eine weitere Substanz angelagert werden, insbesondere wenn der Festphasen-Reaktant aus mehreren, nicht kovalent miteinander verbundenen Komponenten besteht. Zum Aufbringen der zweiten und ggf. weiteren Schichten sind die Reaktionsbedingungen nicht kritisch, so daß ohne Schutzgas und mit Wasser als Lösungsmittel gearbeitet werden kann.

Die nach dem erfindungsgemäßen Verfahren hergestellte Bindeschicht ist mikroskopisch homogen, wie z.B. durch geeignete Verfahren, wie etwa Wellenleiter-Messungen oder Weißlicht-Interferometrie nachweisbar ist.

Ein weiterer Gegenstand der vorliegenden Erfindung ist ein Verfahren zur Herstellung einer kompartimentierten Bindematrix, wobei man räumlich getrennte Bereiche auf einem Trägermaterial, die eine oxidische Oberfläche aufweisen, mit gleichen oder verschiedenen Reaktionslösungen inkubiert, worin Moleküle enthalten sind, die aus oberflächenaktiven Silan-Endgruppen und damit über Spacermoleküle verknüpften, gleichen oder verschiedenen Festphasenreaktanden bestehen. Vorzugsweise werden die räumlich getrennten Bereiche mit oxidischer Oberfläche auf ein Trägermaterial mit nicht-oxidischer Oberfläche, z.B. durch Anlegen einer Maske und Bedampfen oder Besputtern mit oxidischem Material, aufgebracht.

Sollen die Bereiche bzw. Spots der kompartimentierten Bindematrix gleich sein, kann das Trägerelement einfach in eine Reaktionslösung eingetaucht werden, welche die für die Erzeugung der gewünschten Bindematrix notwendigen Moleküle enthält. Sollen die Bindematrices der einzelnen Bereiche jedoch unterschiedlich sein, so werden verschiedene Reaktionslösungen auf einzelne Oxidspots aufgegeben, die jeweils andere Festphasenreaktanden enthalten. Stattdessen oder zusätzlich können auch unterschiedliche Verdünnungsgrade der Bindematrices auf verschiedenen Spots erzeugt werden, indem z.B. ein unterschiedlicher Anteil an Verdünnungsmolekülen in den einzelnen Reaktionslösungen vorhanden ist, die auf verschiedene Spots aufgegeben werden. Diese räumlich getrennte Aufgabe der Reaktionslösungen kann über gebräuchliche Methoden wie Pipettieren, Stempel- oder Drucktechniken, wie z.B. Ink-Jet erfolgen.

Noch ein weiterer Gegenstand der vorliegenden Erfindung ist ein Verfahren zur Herstellung eines Analysenelements, wobei man eine erfindungsgemäße Bindematrix mit mindestens einer Reaktionslösung inkubiert, worin Moleküle von weiteren, an die Bindematrix bindefähigen Reaktionspartnern enthalten sind.

Die Herstellung von kompartimentierten Analysenelementen kann auf verschiedene Weise erfolgen. Im einfachsten Fall wird eine Lösung von gleichen oder verschiedenen Reaktionspartnern auf verschiedene Bindematrix-Spots, die jeweils mit einer verdünnten Bindeschicht belegt sind, z.B. mit einer Pipette aufgebracht. Diese Reaktionspartner müssen zur Bindung an den Festphasenreaktanden an der Oberfläche der kompartimentierten Bindematrix in der Lage sein. Vorzugsweise verwendet man daher als Reaktionspartner Konjugate, die eine zur Bindung an den Festphasenreaktanden fähige Gruppe enthalten. Ein Beispiel für solche Konjugate sind Antikörper, die mit Biotin gekoppelt sind. Auf unterschiedliche Oxid-Spots, die mit einer verdünnten Biotin-Silan-Bindematrix und einer daran gebundenen Streptavidin-Bindeschicht als Festphasenreaktand belegt sind, können auf den einzelnen Spots gleiche oder verschiedene biotinylierte Antikörper aufgebracht werden, die sich dann an die einzelnen Streptavidinschichten binden. Man erhält auf diese Weise ein Analysenelement, das auf engstem Raum verschiedene Felder besitzt, die gegebenenfalls zur Bindung vieler unterschiedlicher freier Analyten in der Lage sind.

Wird auf jeden Spot der gleiche biotinylierte Antikörper aufgebracht, so können mit dem Analysenelement mehrere Proben parallel bestimmt werden, die einen mit dem festphasengebundenen Antikörper reaktiven Analyten enthalten.

Weitere Möglichkeiten zur Aufgabe verschiedener Reaktionspartner auf die jeweiligen Spots sind Druck- oder Stempeltechniken, insbesondere Ink-Jet, oder die Aufgabe mittels einer Mikromultipinplatte analog zur Aufgabe verschiedener Reagenzien auf Mikrotiterplatten.

Weiterhin betrifft die vorliegende Erfindung ein Verfahren zur Bestimmung eines Analyten in einer Probelösung mittels einer spezifischen Bindungsreaktion zwischen mindestens zwei bioaffinen Reaktanden, von denen einer an eine Festphase gekuppelt vorliegt und der oder die anderen Partner frei sind, wobei man einen Festphasen-Reaktanden verwendet, der Bestandteil einer erfindungsgemäßen Bindematrix ist.

Bei einem derartigen Verfahren kann der Nachweis der Bindung des freien Reaktionspartners an den Festphasenreaktanden dadurch ermöglicht werden, daß der freie Bindungspartner eine Markierungsgruppe trägt. Gebräuchlich ist insbesondere die Markierung mit einem Enzym oder mit einem fluoreszierenden oder lumineszierenden Bestandteil. Die dadurch ermöglichte indirekte optische Beobachtung der Bindung erlaubt einen genauen quantitativen Nachweis.

Grundsätzlich kann die Bindung optische, elektrochemisch, aber auch über die Wärmetönung oder die Massenbildung bestimmt werden. Für elektrochemische Techniken kommen insbesondere potentiometrische oder amperometrische Methoden in Frage, wie sie beispielsweise in "Biosensors", Turner, Karube, Wilson (eds.), Oxford Press, 1987 oder Bergveld, Biosensors & Bioelectronics 6, 55-72 (1991) beschrieben sind. Bestimmungen über die elektrische Leitfähigkeit oder Kapazitätsänderung sind als elektrochemische Techniken ebenfalls möglich.

Vorzugsweise erfolgt jedoch der Nachweis der Bindung durch optische, insbesondere reflexionsoptische Techniken, bei der die Zunahme der Schichtdicke einer extrem dünnen Schicht mit dem trägerfixierten Reaktanden durch Bindung des freien Bindungspartners beobachtet werden kann. Ein Überblick über diese Techniken wird gegeben in Sadowski: "Review of optical methods in immunosensing", SPIE, Vol. 954 Optical testing and Metrology II (1988), 413-419.

Ein besonders bevorzugtes reflexionsoptisches Verfahren ist der Nachweis der Bindung durch Wellenleiter-Messungen und Weißlicht-Interferometrie.

Die Wellenleiter-Messungsmethode ist in Anal. Let. 23 (3), 411-424 (1990) beschrieben und beruht darauf, daß Laserlicht über ein Gitter auf dem Sensor in eine wellenleitende Schicht eingekoppelt wird. Die Einkoppelbedingungen (Winkel) sind abhängig von den Verhältnissen an den Grenzflächen der wellenleitenden Schicht. Über die Änderung der Einkoppelbedingungen kann somit auf die Dicke der Bindeschicht auf der Oberfläche geschlossen werden.

Ein weiteres geeignetes Verfahren zur Bestimmung der Schichtdicke an der erfindungsgemäßen Bindematrix ist die Weißlicht-Interferometrie. Derartige Meßsysteme werden von der Firma Carl Zeiss, Oberkochen, BRD vertrieben. Das Meßsystem für die Weißlicht-Interferometrie besteht aus einem Diodenzeilenspektrometer, einer Beleuchtungseinrichtung, vorzugsweise einem Y-Lichtleiter mit 2 Lichtleitbündeln, die an einem Ende zusammengefaßt und statistisch vermischt sind und einer Reflexionsoptik.

Bei Verwendung einer kompartimentierten Bindematrix oder eines kompartimentierten Analysenelements ist die parallele Bestimmung unterschiedlicher, d.h. von mindestens zwei Analyten in einer Probelösung bzw. die parallele Bestimmung eines Analyten in verschiedenen Probelösungen möglich. Ein Gegenstand der vorliegenden Erfindung ist somit ein Verfahren zur parallelen Bestimmung unterschiedlicher Analyten in einer Probelösung, welches dadurch gekennzeichnet ist, daß man die Probelösung mit einer kompartimentierten Bindematrix oder einem kompartimentierten Analysenelement in Kontakt bringt und das Vorhandensein oder/und die Menge der unterschiedlichen Analyten in der Probelösung durch ortsauflösende Messung an den einzelnen räumlich getrennten Bindematrixbereichen bestimmt.

Um die Bindung der Analyten auf einzelnen Spots nebeneinander qualitativ und insbesondere auch quantitativ nachweisen zu können, werden ortsauflösende Detektionsmethoden verwendet. Eine bevorzugte Methode ist die konfokale Fluoreszenzmikroskopie. Bei dieser Methode wird auf jedem Spot einzeln ein zur Markierung verwendeter Fluoreszenzfarbstoff mit Hilfe eines Lasers, der eine geeignete Wellenlänge imitiert, zur Fluoreszenz angeregt. Das emittierte Licht wird mittels empfindlicher Detektoren, wie z.B. integrierender CCD-Kameras, aufgezeichnet und mit einer geeigneten Computersoftware zur Bildauswertung quantifiziert. Auch andere fluoreszenzmikroskopische Methoden sind zur Auswertung gut geeignet. In diesem Fall werden mehrere Spots in einem Bildausschnitt gleichzeitig betrachtet. Die Auswertung der einzelnen Spots erfolgt dann z.B. hintereinander mit Hilfe geeigneter Bildauswertungssoftware.

Weiterhin kann die Auswertung auch mit Hilfe der bereits beschriebenen reflektionsoptischen Techniken erfolgen. Auch andere ortsauflösende Detektionsmethoden, z.B. elektrochemische Methoden, Leitfähigkeitsmessung oder die Messung von radioaktiver Markierungen sind möglich.

Zur Analyse einer Probe auf unterschiedliche Analyten kann die Probe entweder großflächig auf die kompartimentierte Bindematrix bzw. das kompartimentierte Analysenelement oder separat auf einzelne Spots aufgebracht werden. Gegebenenfalls werden zusätzlich markierte Bindungspartner für die zu bestimmenden Analyten zugegeben. In diesem Fall wird das Analysenelement vor Messung der an die Festphase gebundenen, markierten Analyten günstigerweise von nicht gebundenen Markierungsmolekülen frei gewaschen.

Die kompartimentierte Bindematrix bzw. das kompartimentierte Analysenelement kann auch zur parallelen Bestimmung von Analyten in verschiedenen Proben benutzt werden. Gegenstand der Erfindung ist daher auch ein Verfahren zur parallelen Bestimmung eines Analyten in verschiedenen Probelösungen, welches dadurch gekennzeichnet ist, daß man jeweils eine Probelösung mit einzelnen Bereichen einer kompartimentierten Bindematrix oder eines Analysenelements in Kontakt bringt und das Vorhandensein oder/und die Menge des Analyten in jeder Probelösung durch ortsauflösende Messung an den einzelnen, räumlich getrennten Bindematrixbereichen bestimmt. Vorzugsweise erfolgt die Bestimmung über spezifische Bindungsreaktionen des Analyten mit festphasengebundenen Analyt-Bindungspartnern und durch getrennte Messungen der einzelnen Bindematrixbereiche. Die verschiedenen Probelösungen können dabei durch übliche aufgabetechniken wie Pipettieren, Druck- oder Stempeltechniken oder Multipinplatten aufgegeben werden. Auf diese Weise kann auf sehr kleinen Flächen eine Vielzahl von Proben gleichzeitig gemessen werden.

Die erfindungsgemäße Bindematrix bzw. das erfindungsgemäße Analyseelement kann somit hervorragend zum multiplen Analytnachweis, d.g. zum gleichzeitigen Nachweis einer Vielzahl von Analyten bzw. zum gleichzeitigen Nachweis eines Analyten in einer Vielzahl von Proben, insbesondere für Immunoassays, beispielsweise für die Allergiediagnostik, oder für die DNA-Diagnostik, z.B. zum Screenen einer Probe auf Nukleinsäuren mit bestimmten Sequenzen eingesetzt werden.

Als Moleküle, die aus oberflächenaktiven Silan-Endgruppen und einem damit über ein Spacermolekül verknüpften Festphasen-Reaktanden bestehen bzw. als Verdünnermoleküle wird bevorzugt eingesetzt eine Silanverbindung mit einer der allgemeinen Formeln (III) oder (IV)

R¹R²R³Si - Sp - X¹ (III)

oder 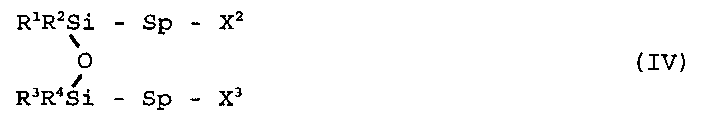 worin R¹, R², R³, R⁴ gleich oder verschieden sein können und Substituenten am Si-Atom bedeuten unter der Voraussetzung, daß mindestens einer der Substituenten R¹, R² und R³ in Formel (III) bzw. mindestens einer der Substituenten R¹ und R² oder R³ und R⁴
in Formel (IV) eine kovalente Bindung mit einer oxidischen Oberfläche eingehen kann,
Sp ein flexibles, lineares Spacermolekül mit einer Kettenlänge von 2 bis 50 Atomen ist, und
X¹, X² und X³ kovalent an das Spacermolekül gebundener Festphasenreaktanden oder/und C₁-C₄-Alkoxy(oligo-C₂-C₄alkylenoxid)gruppen sind.

Vorzugsweise sind die Reste R¹, R², R³ und R⁴ in den Silangruppen der Verbindungen (III) und (IV) C₁-C₄-Alkylgruppen, C₁-C₄-Alkoxygruppen, Hydroxygruppen, Aminogruppen oder Halogenatome. Besonders bevorzugt sind R¹, R², R³ und R⁴ C₁-C₂-Alkoxygruppen und am meisten bevorzugt Methoxygruppen.

Wenn X¹, X² und X³ Festphasenreaktanden bedeuten, sind sie vorzugsweise Biotin oder ein Biotinderivat. Sp umfaßt vorzugsweise eine gegebenenfalls substituierte oder/und Heteroatome enthaltende Alkylengruppe.

Die unspezifische Bindung von Proteinen, z.B. Fibrinogen, an oxidische Oberflächen kann überraschenderweise durch kovalente Bindung der Silane, insbesondere mit Methoxyoligoethylenglykol-Endgruppen, an die Oberfläche unterdrückt werden.

Die vorliegende Erfindung soll im weiteren durch die folgenden Beispiele verdeutlicht werden.

### BEISPIEL 1

### Synthese von Biotinsilan 1

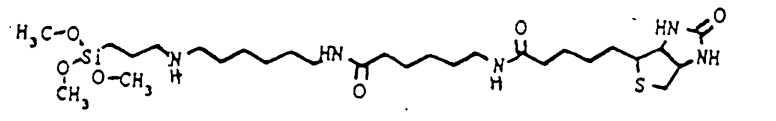

2,0 g Biotinoyl-ε-aminocapronsäure-N-hydroxysuccinimidester (Biotin-X-OSu) wurden in 40 ml Methanol suspendiert. 1,0 g 6-(Aminohexylaminopropyl)-trimethoxysilan wurde zugetropft und die Lösung 1 1/2 Stunden bei Raumtemperatur gerührt. Das Methanol wurde anschließend abdestilliert. Das Produkt wurde anschließend säulenchromatographisch gereinigt. Es wurden 1,3 g eines weißen Feststoffs erhalten.

### BEISPIEL 2

### Synthese von Biotinsilan 2

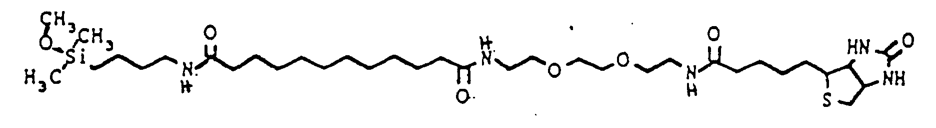

### 2.1 Synthese von Biotin-DADOO-Dodecansäure-OSu-Ester

560 mg Biotin-DADOO wurde in 30 ml DMF gelöst und zusammen mit 150 ml Triethylamin in eine Lösung von 6,36 g Dodecansäurebis-OSu-Ester getropft. Die Lösung wurde 3 Stunden bei Raumtemperatur gerührt, anschließend das Lösungsmittel weitgehend abgezogen und der Rückstand über Sephadex® chromatographiert.

### 2.2 Synthese von Biotinsilan 2

280 mg des isolierten Biotin-DADOO-dodecansäure-OSu-esters wurden in 5 ml Methanol gelöst. 80 mg Aminobutyldimethylmethoxysilan wurde zur Lösung getropft und 1 h gerührt. Die Lösung wurde anschließend mit 30 ml Diethylether versetzt und der ausgefallene Feststoff abgesaugt. Die Aufreinigung erfolgte chromatographisch über NH₂-Kieselgel. Die Ausbeute betrug 50 mg.

### BEISPIEL 3

### Synthese des Biotinsilans 3

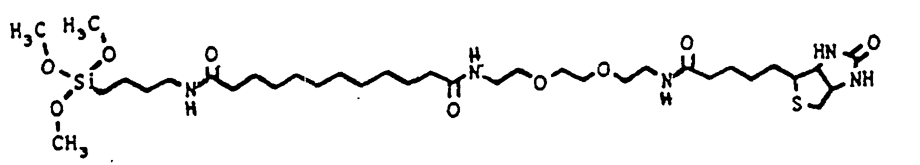

680 mg des Biotin-DADOO-dodecansäure-OSu-esters wurden in 7 ml Methanol gelöst. 215 mg Aminopropyltrimethoxysilan wurden zur Lösung getropft und 1 h gerührt. Die Lösung wurde anschließend über eine NH₂-Kieselgelsäule aufgereinigt. Die Produktfraktionen wurden eingeengt, mit 50 ml Diethylether versetzt und der ausgefallene Feststoff abgesaugt. Die Ausbeute betrug 420 mg.

### BEISPIEL 4

### Synthese des Silanverdünners 4

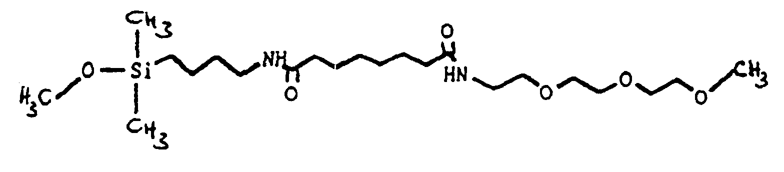

### 4.1 Synthese von Triethylenglykolmonomethylether-tosylat

Zu einer Lösung von 53,2 ml Triethylenglykolmonoethylether in 400 ml Pyridin wurden bei -5°C langsam 69,6 g Tosylchlorid zugegeben. Es wurde 3 h bei 0°C und 1 h bei Raumtemperatur gerührt. Der Ansatz wurde mit 400 ml Wasser versetzt und anschließend mit Essigester extrahiert. Aus der Essigesterphase wurde 90 g des Tosylats (85 % Ausbeute) isoliert.

### 4.2 Synthese von Triethylenglykolmonomethylether-phthalimid

9,55 g des Tosylats wurden in 20 ml DMF gelöst und zu einer Lösung von 6,11 g Phthalimid-Kaliumsalz in 80 ml DMF getropft. Die Mischung wurde 4 h bei 120°C gerührt, anschließend mit Wasser verdünnt und mit Essigester extrahiert. Aus der Essigesterphase wurden 4,7 g des Produkts in Form eines Öls isoliert (53 %).

### 4.3 Synthese von 1-Amino-triethylenglykolmonomethylether

1,53 g des unter 4.2 isolierten Produkts wurden in 25 ml Methanol gelöst. 0,32 ml 80 %ige Hydrazinhydratlösung wurden zugetropft und 3 h bei 50°C und anschließend 15 min bei Rückfluß gekocht. Die Mischung wurde anschließend mit HCl angesäuert und nach dem Abkühlen filtriert. Das Filtrat wurde neutralisiert und anschließend mit Chloroform extrahiert. Aus der Chloroformphase wurden 740 mg Produkt 1-Amino-triethylenglykolmonomethylether in Form eines Öls isoliert (87 % Ausbeute).

### 4.4 Synthese von Korksäure-1-OSu-Ester-12-amidotriethylenglykolmonomethylester

15,8 g Korksäurebis-OSu-ester wurden zusammen mit 0,6 ml Triethylamin in 20 ml DMF gelöst. Zu dieser Lösung wurden 700 mg des unter 4.3 isolierten Produkts in 10 ml DMF gegeben. Der Ansatz wurde 4 h bei Raumtemperatur gerührt und anschließend das DMF abgezogen. Der Rückstand wurde mit 20 ml Wasser versetzt und anschließend filtriert. Das Filtrat wurde vom Lösungsmittel befreit und anschließend über eine Kieselgelsäule gereinigt. Man erhielt das Produkt als Öl in einer Ausbeute von 750 mg (42 %).

### 4.5 Synthese von 4

270 mg des Produkts aus 4.4 wurden in einem Gemisch aus 5 ml Diethylether und 1 ml Methanol gelöst. 0,09 ml Triethylamin wurde zugegeben und anschließend 115 mg Aminobutyl-dimethylmonomethoxysilan zugetropft. Es wurde 1 h bei Raumtemperatur gerührt und das Produkt anschließend chromatographisch gereinigt. Vom Silanverdünner 4 wurden 190 mg (63 %) erhalten.

### BEISPIEL 5

### 5.1 Synthese des Silanverdünners 5

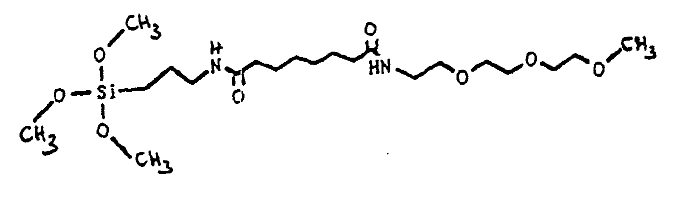

270 mg des Produkts aus 4.4 wurden in einem Gemisch aus 5 ml Diethylether und 1 ml Methanol gelöst. 0,09 ml Triethylamin wurde zugegeben und anschließend 127 mg Aminobutyl-trimethoxysilan zugetropft. Es wurde anschließend 1 h bei Raumtemperatur gerührt und das Produkt dann chromatographisch gereinigt. Vom Silanverdünner 5 wurden 190 mg (61 %) erhalten.

### 5.2 Synthese des Silanverdünners 6

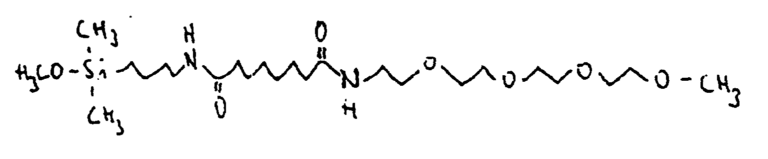

Die Synthese dieses Silans verläuft völlig analog der bereits vorstehend beschriebenen Synthese von 4. Anstelle von Triethylenglycolmonomethylether wurde als Ausgangsstubstanz Tetraethylenglycolmonomethylether eingesetzt.

### 5.3 Synthese des Silanverdünners 7

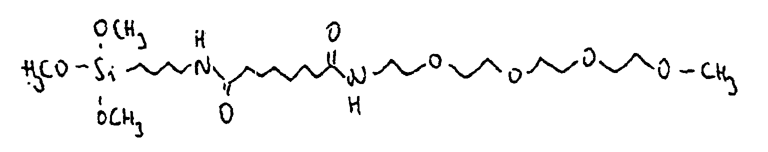

Die Synthese dieses Silans verläuft völlig analog der bereits beschriebenen Synthese von 5. Anstelle von Trethylenglycolmonomethylether wird Tetraethylenglycolmonomethylether als Ausgangssubstanz eingesetzt.

### BEISPIEL 6

Weiterhin wurden folgende Silanverbindungen hergestellt:

### 6.1 Biotinsilanverbindung 8

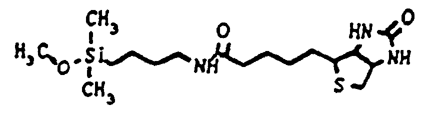

### 6.2 Biotinsilanverbindung 9

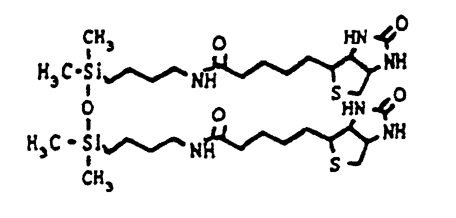

### 6.3 Biotinsilanverbindung 10

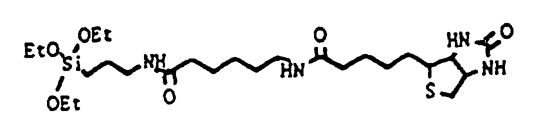

### 6.4 Biotinsilanverbindung 11

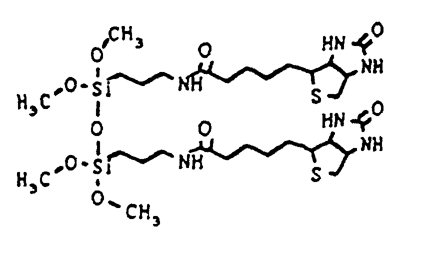

### 6.5 Biotinsilanverbindung 12

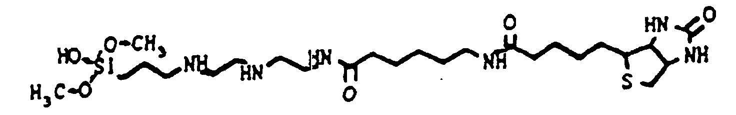

### 6.6 Biotinsilanverbindung 13

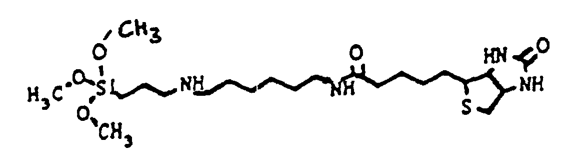

1,7 g Biotin-X-OSu wurden in 20 ml Methanol suspendiert und dazu wurden 1,28 g 6-(Aminohexylaminopropyl)-trimethoxysilan zugetropft. Die Lösung wurde 1,5 Stunden gerührt und anschließend über eine Amino-Kieselgelsäule chromatographiert. Es wurden 2,1 g eines weißen Feststoffs erhalten.

### 6.7 Biotinsilanverbindung 14

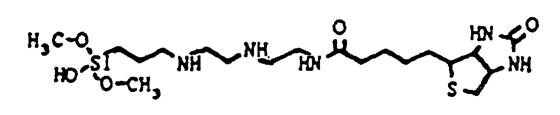

### 6.8 Biotinsilanverbindung 15

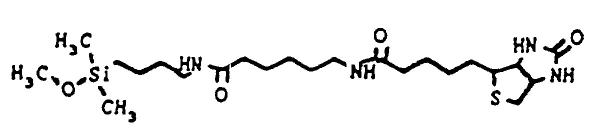

850 mg Biotin-X-OSu wurden in 10 ml Methanol suspendiert und zu dieser Suspension wurden 280 mg 4-Aminobutyldimethylmethoxysilan getropft. Die Lösung wurde 4 Stunden bei Raumtemperatur gerührt und anschließend über eine Amino-Kieselgelsäule aufgereinigt. Es wurden 700 mg eines weißen Feststoffs erhalten.

### 6.9 Biotinsilanverbindung 16

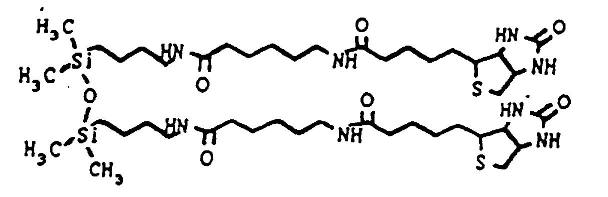

### 6.10 Biotinsilanverbindung 17

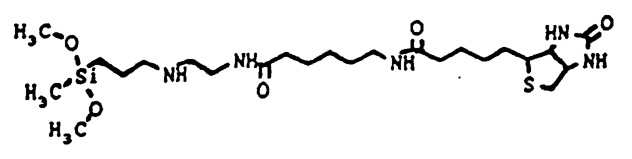

800 mg Biotin-X-OSu wurden in 20 ml Methanol suspendiert. Zu dieser Suspension wurden 300 mg (2-Aminoethylamino)propylmethyldimethoxysilan getropft und 2,5 Stunden gerührt. Der Ansatz wurde anschließend über eine Amino-Kieselgelsäule gereinigt. Es wurden 650 mg an Produkt erhalten.

### 6.11 Biotinsilanverbindung 18

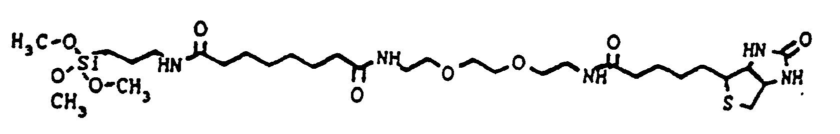

### 6.12 Biotinsilanverbindung 19

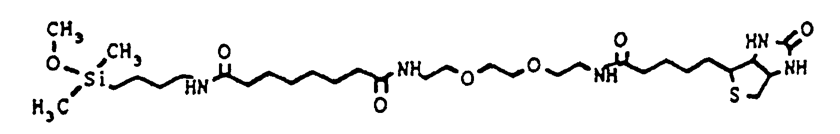

### 6.13 Biotinsilanverbindung 20

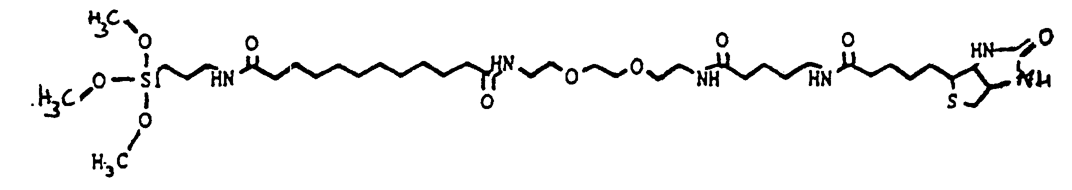

### 6.14 Biotinsilanverbindung 21

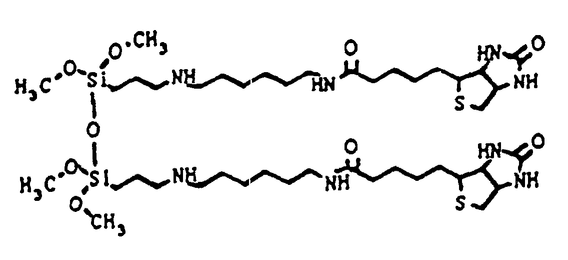

### 6.15 Silanverdünner 22

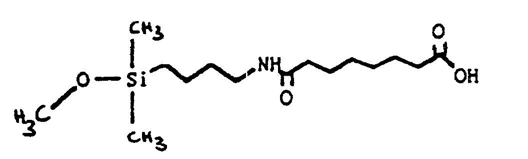

### 6.16 Silanverdünner 23

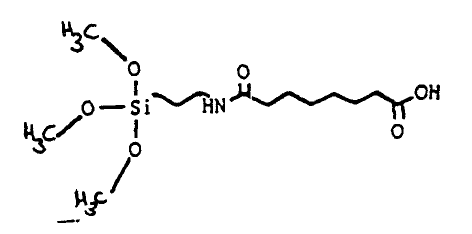

### BEISPIEL 7

### Methoden zur Bewertung der Oberflächen

### 7.1 Wellenleiter-Messungen

Die Messungen wurden auf Sensoren und Gerät der Firma ASI (Artificial Sensing Instruments, Zürich) durchgeführt. Bei der Messung wird Laserlicht über ein Gitter auf dem Sensor in eine wellenleitende Schicht eingekoppelt. Die Einkoppelbedingungen (Winkel) sind abhängig von den Verhältnissen an den Grenzflächen der wellenleitenden Schicht. Über die Änderung der Einkoppelbedingungen kann somit auf die Schichtdicken der aufwachsenden Schichten geschlossen werden. Die Messungen wurden in Durchflußküvetten durchgeführt. Bei der wellenleitenden Schicht handelt es sich um ein Mischoxid aus SiO₂/TiO₂.

### 7.2 Weißlicht-Interferometrie (WIF)

Die Messungen wurden auf einem Zeiss-Meßplatz mit einer Zeiss CLX-111 Weißlichtquelle und einem Zeiss MCS-310 Diodenarray-Spektrometer als Detektoreinheit durchgeführt. Als Sensoren wurden Schott-Farbfiltergläser WG 345 verwendet, die in einer Leybold Beschichtungsanlage Univex 450 mit ca. 800 nm SiO₂ beschichtet wurden. Die Messungen wurden in Durchflußküvetten durchgeführt. Aus den erhaltenen Interferogrammen können die Schichtdicken ermittelt werden.

### BEISPIEL 8

### Aufbringen der Biotinsilane auf oxidische Oberflächen

### 8.1 Vorbereitung der Oberflächen

Die zu beschichtenden oxidischen Oberflächen werden einige Minuten in Königswasser gekocht, anschließend 3 x mit bidestilliertem Wasser gewaschen und bei 80°C 1 Stunde im Vakuum getrocknet.

### 8.2 Unvollständige Silanisierung der Oberflächen zur Erzeugung einer verdünnten Biotinschicht ohne Verwendung eines Silan-Verdünner.

Das Silan 1 wird in einer Konzentration von 10⁻³ Gew.-% in trockenem Methanol gelöst. Die zu beschichtende und gemäß 8.1 vorbereitete Probe wird in diese Lösung getaucht und 30 Minuten bei Raumtemperatur in der Lösung belassen. Die Oberflächen werden anschließend 3 x mit trockenem Methanol abgespült und 30 Minuten bei 80°C im Vakuum konditioniert.

### 8.3 Verfahren zur Beschichtung oxidischer Oberflächen mit Biotinsilanen, hydrophilen Verdünnern oder Mischschichten aus beiden.

Die Silane bzw. deren Mischungen werden in einer Gesamtkonzentration von 1 Gew.-% jeweils entsprechend ihren molaren Anteilen in den Mischungen in trockenem Methanol gelöst. Die zu beschichtende und gemäß 8.1 vorbereitete Probe wird in einem geschlossenen Gefäß unter Methanol-gesättigter Intertgasatmosphäre mit dieser Lösung bedeckt. Die Reaktionszeit beträgt 4 Stunden bei Raumtemperatur. Die Oberfläche wird anschließend mit Methanol abgespült und die Probe 4 Stunden bei 120°C im Trockenschrank konditioniert.

### BEISPIEL 9

### Ergebnisse der Bewertungen (Weißlichtinterferometrie)

Die Proteinlösungen wurden bei Weißlichtinterferometrie-Messungen mittels einer Pharmacia Peristaltik-Pumpe durch eine Teflon-Küvette gepumpt. Die Zyklen betrugen jeweils 10 min mit 5 min-Intervallen zur Zwischenspülung (d.h. 10 min Puffer, 10 min Proteinlösung 1, 5 min Puffer, 10 min Proteinlösung 2, etc.).

### 9.1 Verwendete Lösungen

Puffer: PBS-Puffer (50 mM Kaliumphosphat, 150 mM NaCl, pH 7,2)
Streptavidin-Lösung: c = 0,5 mg/ml in PBS
Biotin-<HCG>-Fab-Fragment
(Bi-Fab): c = 0,1 mg/ml in PBS
Humanchoriongonadotropin
(HCG): c = 5 µg/ml
Rinder-Fibrinogen: c = 3 mg/ml

### 9.2 Bindung von Fibrinogen

Gemessen mit WIF, 10 Minuten PBS-Puffer, 10 Minuten Fibrinogenlösung, 10 Minuten PBS-Puffer

### 9.2.1 Einheitlich funktionalisiserte Oberflächen (keine gemischte Schicht).

Alle Silane außer die Fluorsilane und Trichloroctadecylsilan wurden gemäß 8.3 aufgebracht. Für die Fluorsilane und Trichloroctadecylsilan wurde als Lösungsmittel Chloroform verwendet.

| Oberfläche | Schichtdicke (nm) |
|---|---|
| unbehandeltes SiO₂ | 4,0 |
| SiO₂ + Silanverdünner 5 | 0,0 |
| SiO₂ + Silanverdünner 4 | 2,0 |
| SiO₂ + Silanverdünner 6 | 2,0 |
| SiO₂ + Silanverdünner 7 | 0,0 |
| SiO₂ + Biotinsilan 1 | 4,1 |
| SiO₂ + Biotinsilan 2 | 3,8 |
| SiO₂ + 3-Aminopropyltrimethoxysilan (Aldrich) | 3,5 |
| SiO₂ + N-[(Trimethoxylsilyl)-propyl]ethylendiamintriessigsäure-Na₃ Salz (ABCR) | |
| pH 7,2 | 1,3 |
| pH 9,0 | 1,2 |
| pH 3,0 | 7,5 |
| SiO₂ + 1H,1H,2H,2H-Perfluordecyltrichlorsilan (ABCR) | 2,6 |
| SiO₂ + 1H,1H,2H,2H-Perfluordecyldimethylchlorsilan (ABCR) | 2,0 |
| SiO₂ + Trichloroctadecylsilan (Merck) | 3,5 |

### 9.2.2 Oberflächen mit gemischter Schicht

| | Biotinsilan 1/ Verdünner 5 Schichtdicke | Biotinsilan 2/ Verdünner 4 Schichtdicke | Biotinsilan 1/ Verdünner 7 Schichtdicke |
|---|---|---|---|
| X_{B} | (nm) | (nm) | (nm) |
| 0,0 | 0,0 | 2,0 | 0,0 |
| 0,05 | 0,6 | 1,2 | 0,0 |
| 0,1 | 0,6 | 2,0 | 0,0 |
| 0,2 | 0,0 | 2,0 | 0,0 |
| 0,5 | 0,7 | 2,3 | 2,3 |
| 1,0 | 4,1 | 3,8 | 4,1 |
| X_{B} = Molenbruch der Biotinkomponente | | | |

### 9.3 Spezifische Bindung von Streptavidin

| | Biotinsilan 1/ Verdünner 5 Schichtdicke | Biotinsilan 2/ Verdünner 4 Schichtdicke | Biotinsilan 1 Verdünner 7 Schichtdicke |
|---|---|---|---|
| X_{B} | (nm) | (nm) | (nm) |
| 0,0 | 0,0 | 1,1 | 0,0 |
| 0,05 | 1,8 | 0,9 | 1,0 |
| 0,1 | 3,8 | 1,2 | 0,5 |
| 0,2 | 6,0 | 1,7 | 0,5 |
| 0,5 | 4,9 | 2,9 | 1,0 |
| 1,0 | 4,5 | 3,4 | 2,6 |

### 9.4 Unspezifische Bindung von Streptavidin

Mit Biotin gesättigtes Streptavidin wurde über die Sensoroberfläche geleitet.

| Sensor | Schichtdickenzunahme (nm) |
|---|---|
| Verdünner 5/Biotinsilan 1 (X_{B} = 0,2) | 0,0 |
| Verdünner 5/Biotinsilan 1 (X_{B} = 0,5) | 0,0 |
| Verdünner 4/Biotinsilan 2 (X_{B} = 0,5) | 1,5 |

### 9.5 Durchführung von HCG-Immuntests

Mit Streptavidin beladene Sensoren wurden mit einem biotinyliertem <HCG> Fab-Fragment beladen. Anschließend wurde HCG-Lösung über den Sensor geleitet.

| Sensor | Schichtdicken (nm) | | |
|---|---|---|---|
| | Streptav. | Bi-Fab | HCG |
| Verdünner 5/Biotinsilan 1 X_{B} = 0,1 | 3,8 | 1,7 | 0,4 |
| Verdünner 5/Biotinsilan 1 X_{B} = 0,2 | 6,0 | 2,3 | 0,6 |

### BEISPIEL 10

### Ergebnisse der Bewertung mit Wellenleiter-Technologie (ASI)

Die ASI-Sensoren wurden mit den Biotinsilanverbindungen 1, 13, 15 und 17 beschichtet. Die Beschichtung erfolgte ohne die Verwendung eines Verdünners gemäß dem unter Punkt 8.2 beschriebenen Verfahren. Bewertet wurde das Bindevermögen von Streptavidin an diese Sensoren sowie der Anteil an unspezifisch gebundenem Streptavidin.

Die Schichtdicke der Sensoren war nach der Beladung mit Biotinsilanen geringer als vor der Beladung (zwischen 0,01 und 0,81 nm). Die Einkopplungswinkel (TM+) und (TE+) nahmen durch die Beschichtung ebenfalls ab.

Der Anteil der unspezifischen Bindung des Streptavidins an die silanisierte ASI-Sensoroberfläche bezüglich der gesamten Streptavidinbindung war je nach Biotinsilanverbindung recht unterschiedlich:

| | |
|---|---|
| 17 | 24 % |
| 15 | 73 % |
| 1 | 9 % |
| 13 | 20 % |
| MeOH | 91 % |

17, 1 und 13 binden das Streptavidin gut (→ kein Abbluten).

Der Anteil der unspezifischen Bindung von Bi-MAK<TSH> (in Anwesenheit von Biotin) an eine mit Biotin abgesättigte Streptavidinoberfläche war für alle Biotinsilane ebenfalls unterschiedlich:

| | |
|---|---|
| 17 | 20 % |
| 15 | < 100 % |
| 1 | 42 % |
| 13 | 31 % |
| MeOH | 24 % |

17 war die beste der untersuchten Biotinsilan-Verbindungen. Mit 17 behandelte Sensoren ergeben den höchsten Signalhub beim Analyten (TSH).

### BEISPIEL 11:

### Verwendung einer mikrokompartimentierten Bindematrix in einem Immunoassay

### 11.1 Herstellung von SiO₂-Spots durch Aufdampfen

Auf"Lexan®" Polycarbonatfolien (Hersteller: General Electric, Dicke: 0,75 mm) mit den Abmessungen 8 x 8 cm wurden über eine Metallmaske (d = 0,5 mm, Material Aluminium) in einer Leybold Hochvakuum-Beschichtungsanlage (Univex 450) mittels Elektronenstrahlverdampfung 36 x 36 runde Spots aus SiO₂ (insgesamt 1296 Spots) in gleichmäßigen Abständen aufgedampft. Die Schichtdicke des aufgedampften Oxids beträgt 150 nm. Der Durchmesser der Spots beträgt 1 mm, der Abstand von Spot zu Spot beträgt ebenfalls 1 mm nach allen Seiten.

### 11.2 Aufbringen der Biotinsilane auf die Spots

Eine Mischung aus Silan 1 und Silan 5 im molaren Verhältnis 1/9 wurde in einer Gesamtkonzentration von 1 Gew.-% in Methanol gelöst. Zu dieser Lösung wurde 1% Wasser zugegeben und das mit Substrat mit den Spots in diese Lösung getaucht. Die Reaktionszeit betrug 4 Stunden. Nach dieser Zeit wurde die Platte mit Methanol gewaschen und 4 Stunden bei 120°C getrocknet.

### 11.3 Aufbringen von Streptavidin

Die Platten mit den auf diese Weise beschichteten Spots wurden eine Stunde mit einer Streptavidin-Lösung in 50 mmol/l K-Phosphat-Puffer, pH 7,2 überschichtet (c= 0,5 mg/ml). Die Platte wurde danach mit einer Lösung aus 50 mmol/l K-Phosphat-Puffer pH 7,2, 2% Saccharose, 0,9% NaCl und 0,3% Rinderserumalbumin II gewaschen und anschließend im Klimaschrank bei 25°C und 40% Luftfeuchtigkeit 20 Stunden getrocknet.

### 11.4 Durchführung eines TSH-Tests

Auf den mit Streptavidin beschichteten Spots wurde ein TSH-Test nach dem folgenden Schema durchgeführt:
1. Inkubation mit dem biotinylierten monoklonalen Anti-TSH-Antikörper 1.20-Bi (0Su) 1:8 für 45 Minuten. Die Konzentration des Antikörpers war 10 µg/ml, Puffer: 20 mmol/l K-Phosphat pH 7,4.
2. Waschen mit Puffer
3. Minuten Inkubation mit Standards "Enzymun®", Fa. Boehringer Mannheim bzw. mit einer Plasmaprobe.
4. Waschen mit Puffer
5. Minuten Inkubation mit Fluoreszenz-Latex-Antikörper (A8)-Konjugat (Charge DF499), 0,02% Feststoffanteil in Puffer.
6. Waschen mit Puffer
7. Messung in einem konfokalen Fluoreszenzmikroskop (Fa. Biorad)

### 11.5 Ergebnisse:

Es wurden 2 Standards (0 µU und 2,3 µU sowie eine Plasmaprobe mit 3,2 µU TSH) vermessen. Dabei wurden die folgenden relativen Intensitäten erhalten:

| Probe | rel. Intensitäten |
|---|---|
| | |
| 0 µU TSH | 26,4 |
| 2,3 µU TSH | 112,7 |
| Plasma (3,2 µU TSH) | 186,7 |

## Patentansprüche

1. Bindematrix, enthaltend ein Trägermaterial mit einer oxidischen Oberfläche und einen daran über Ankergruppen kovalent gebundenen Festphasen-Reaktanten, der mit mindestens einem freien Reaktionspartner bindefähig ist,
**dadurch gekennzeichnet,**
**dass** der Festphasen-Reaktant eine verdünnte und im Wesentlichen lateral homogene Bindeschicht auf der Oberfläche des Trägermaterials bildet und dass die Ankergruppen Silangruppen sind und mit dem Festphasenreaktanten über ein Spacermolekül verknüpft sind und dass die Bindematrix neben den mit Festphasen-Reaktanten verknüpften Spacermolekülen noch Verdünnermoleküle enthält, die mit Ankergruppen kovalent an die Festphase gebunden sind.

2. Bindematrix nach Anspruch 1,
**dadurch gekennzeichnet,**
**dass** die Belegung des Festphasen-Reaktanten auf der Oberfläche des Trägermaterials von 0,1 bis 90 % der maximalen Belegung ist.

3. Bindematrix nach Anspruch 2,
**dadurch gekennzeichnet,**
**dass** die Belegung des Festphasen-Reaktanten auf der Oberfläche des Trägermaterials von 0,5 bis 70 % der maximalen Belegung ist.

4. Bindematrix nach Anspruch 3,
**dadurch gekennzeichnet,**
**dass** die Belegung des Festphasen-Reaktanten auf der Oberfläche des Trägermaterials von 1 bis 40 % der maximalen Belegung ist.

5. Bindematrix nach einem der Ansprüche 1 bis 4,
**dadurch gekennzeichnet,**
**dass** die Ankergruppen durch Reaktion der oxidischen Trägermaterialoberfläche mit einer oberflächenreaktiven Silangruppe der allgemeinen Formel (I) oder/und (II) gebildet sind:
R¹R²R³Si - (I)
oder 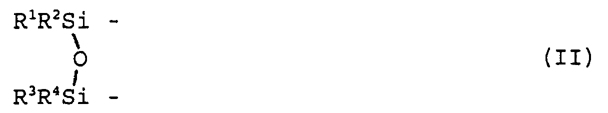 worin R¹, R², R³, R⁴ gleich oder verschieden sein können und Substituenten am Si-Atom bedeuten, unter der Voraussetzung, daß mindestens einer der Substituenten R¹, R² und R³ in Formel (I) bzw. mindestens einer der Substituenten R¹ und R² oder R³ und R⁴ in Formel (II) eine kovalente Bindung mit einer oxidischen Oberfläche eingehen kann.

6. Bindematrix nach Anspruch 5,
**dadurch gekennzeichnet,**
**dass** die Substituenten R¹, R², R³, R⁴ in den Silangruppen der Formeln (I) und (II) C₁-C₄-Alkyl-, C₁-C₄-Alkoxy-, Hydroxy-, Aminogruppen oder/und Halogenatome sind.

7. Bindematrix nach einem der Ansprüche 5 oder 6,
**dadurch gekennzeichnet,**
**dass** alle Substituenten R¹, R², R³, R⁴ eine kovalente Bindung mit der oxidischen Oberfläche eingehen können.

8. Bindematrix nach einem der Ansprüche 1 bis 7,
**dadurch gekennzeichnet,**
**dass** die Ankergruppe mit dem Festphasen-Reaktanten über ein flexibles, lineares Spacermolekül verknüpft ist, das eine Kettenlänge von 2 bis 50 Atomen aufweist.

9. Bindematrix nach Anspruch 8,
**dadurch gekennzeichnet,**
**dass** das flexible Spacermolekül eine gegebenenfalls substituierte oder/und Heteroatome enthaltende Alkylengruppe umfasst.

10. Bindematrix nach einem der Ansprüche 1 bis 9,
**dadurch gekennzeichnet,**
**dass** sich zwischen dem Spacermolekül und dem Festphasen-Reaktanten eine hydrophile Linkergruppe befindet.

11. Bindematrix nach Anspruch 10,
**dadurch gekennzeichnet,**
**dass** die hydrophile Linkergruppe eine oder mehrere Oxyethylengruppen enthält.

12. Bindematrix nach Anspruch 11,
**dadurch gekennzeichnet,**
**dass** die hydrophile Linkergruppe durch ein Amin- oder Hydroxyl-terminiertes Polyethylenoxid gebildet wird.

13. Bindematrix nach Anspruch 12,
**dadurch gekennzeichnet,**
**dass** die hydrophile Linkergruppe aus 1,8-Diamino-3,6-dioxaoctan gebildet ist.

14. Bindematrix nach einem der Ansprüche 1 bis 13,
**dadurch gekennzeichnet,**
**dass** die Verdünnermoleküle eine Alkoxy(oligoalkylenoxid)-Endgruppe aufweisen.

15. Bindematrix nach einem der vorhergehenden Ansprüche,
**dadurch gekennzeichnet,**
**dass** der Festphasen-Reaktant ein mit einem Antikörper bindefähiges Antigen oder Hapten ist.

16. Bindematrix nach einem der Ansprüche 1 bis 14,
**dadurch gekennzeichnet,**
**dass** der Festphasen-Reaktant Biotin oder ein Biotinderivat ist.

17. Bindematrix nach einem der Ansprüche 1 bis 14,
**dadurch gekennzeichnet,**
**dass** der Festphasen-Reaktant aus einer inneren und einer äußeren Komponente besteht, wobei die äußere Komponente mit mindestens einem freien Reaktionspartner bindefähig ist.

18. Bindematrix nach Anspruch 17,
**dadurch gekennzeichnet,**
**dass** die innere Komponente des Festphasen-Reaktanten eine unverdünnte Schicht auf der Oberfläche des Trägermaterials bildet und die äußere Komponente an die innere Komponente durch Affinitätsbindung gekoppelt ist.

19. Bindematrix nach Anspruch 18,
**dadurch gekennzeichnet,**
**dass** die innere Komponente Biotin und die äußere Komponente Streptavidin ist.

20. Kompartimentierte Bindematrix,
**dadurch gekennzeichnet,**
**dass** auf einem Trägermaterial eine Vielzahl von räumlich getrennten Bereichen angeordnet ist, die jeweils eine Bindematrix nach einem der Ansprüche 1 bis 19 umfassen.

21. Kompartimentierte Bindematrix nach Anspruch 20,
**dadurch gekennzeichnet,**
**dass** die räumlich getrennten, jeweils eine Bindematrix umfassenden Bereiche auf einem Trägermaterial mit nicht-oxidischer Oberflächeangeordnet sind.

22. Kompartimentierte Bindematrix nach Anspruch 20 oder 21,
**dadurch gekennzeichnet,**
**dass** die räumlich getrennten Bereiche Bindematrices mit jeweils gleichen Festphasen-Reaktanten umfassen.

23. Kompartimentierte Bindematrix nach Anspruch 20 oder 21,
**dadurch gekennzeichnet,**
**dass** die räumlich getrennten Bereiche Bindematrices mit unterschiedlichen Festphasen-Reaktanten umfassen.

24. Analysenelement, umfassend eine verdünnte Bindematrix nach einem der Ansprüche 1 bis 23,
**dadurch gekennzeichnet,**
**dass** der Festphasenreaktant mit einer oder mehreren zusätzlichen Schichten von Reaktionspartnern belegt ist.

25. Analysenelement nach Anspruch 24,
**dadurch gekennzeichnet,**
**dass** die Reaktionspartner aus Antigenen, Haptenen, Antikörpern oder Nukleinsäuren ausgewählt werden.

26. Analysenelement nach Anspruch 24 oder 25 zur Bestimmung unterschiedlicher Analyten in einer Probe, umfassend eine kompartimentierte Bindematrix nach Anspruch 20, worin die einzelnen räumlich getrennten Bindematrix-Bereiche mit jeweils unterschiedlichen weiteren Reaktionspartnern belegt sind.

27. Analysenelement nach Anspruch 24 oder 25 zur Bestimmung gleicher Analyten in verschiedenen Proben, umfassend eine kompartimentierte Bindematrix nach Anspruch 20, worin die einzelnen räumlich getrennten Bindematrix-Bereiche mit jeweils gleichen Reaktionspartnern belegt sind.

28. Verfahren zur Bestimmung eines Analyten in einer Probelösung mittels einer spezifischen Bindungsreaktion zwischen mindestens zwei bioaffinen Reaktanten, von denen einer an eine Festphase gekoppelt vorliegt und der oder die anderen Reaktionspartner frei sind,
**dadurch gekennzeichnet,**
**dass** man einen an die Festphase gekoppelten Reaktanten verwendet, der Bestandteil einer Bindematrix nach einem der Ansprüche 1 bis 23 oder eines Analysenelements nach einem der Ansprüche 24 bis 27 ist.

29. Verfahren nach Anspruch 28,
**dadurch gekennzeichnet,**
**dass** man die spezifische Bindungsreaktion durch Wellenleiter-Messungen oder durch Weißlicht-Interferometrie bestimmt.

30. Verfahren nach Anspruch 28 zur parallelen Bestimmung unterschiedlicher Analyten in einer Probelösung,
**dadurch gekennzeichnet,**
**dass** man die Probelösung mit einer kompartimentierten Bindematrix nach Anspruch 23 oder einem Analysenelement nach Anspruch 26 in Kontakt bringt und das Vorhandensein oder/und die Menge der unterschiedlichen Analyten in der Probelösung durch ortsauflösende Messung an den einzelnen räumlich getrennten Bindematrix-Bereichen bestimmt.

31. Verfahren nach Anspruch 28 zur parallelen Bestimmung eines Analyten in verschiedenen Probelösungen,
**dadurch gekennzeichnet,**
**dass** man jeweils eine Probelösung mit einzelnen Bereichen einer kompartimentierten Bindematrix nach Anspruch 22 oder eine Analysenelements nach Anspruch 27 in Kontakt bringt und das Vorhandensein oder/und die Menge des Analyten in jeder Probelösung durch ortsauflösende Messung an den einzelnen, räumlich getrennten Bindematrix-Bereichen bestimmt.

32. Verfahren nach Anspruch 30 oder 31,
**dadurch gekennzeichnet,**
**dass** die ortsauflösende Messung mittels konfokaler Fluoreszenzmikroskopie erfolgt.

33. Verfahren zur Herstellung einer Bindematrix nach einem der Ansprüche 1 bis 19,
**dadurch gekennzeichnet,**
**dass** man ein oxidisches Trägermaterial mit einer Reaktionslösung inkubiert, worin Moleküle enthalten sind, die aus oberflächenaktiven Silan-Endgruppen und einem damit über ein Spacermolekül verknüpften Festphasen-Reaktanten bestehen, wobei die Reaktionslösung noch zusätzlich Verdünnermoleküle enthält, welche Ankergruppen umfassen, die kovalent an die Festphase gebunden werden.

34. Verfahren zur Herstellung einer kompartimentierten Bindematrix nach einem der Ansprüche 20 bis 23,
**dadurch gekennzeichnet,**
**dass** man räumlich getrennte Bereiche auf einem Trägermaterial, die eine oxidische Oberfläche aufweisen, mit gleichen oder verschiedenen Reaktionslösungen inkubiert, worin Moleküle enthalten sind, die aus oberflächenaktiven Silan-Endgruppen und damit über Spacermoleküle verknüpften gleichen oder verschiedenen Festphasenreaktanten bestehen.

35. Verfahren nach Anspruch 34,
**dadurch gekennzeichnet,**
**dass** die räumlich getrennten Bereiche auf ein Trägermaterial mit nicht-oxidischer Oberfläche aufgebracht werden.

36. Verfahren zur Herstellung eines Analysenelements nach einem der Ansprüche 24 bis 27,
**dadurch gekennzeichnet,**
**dass** man eine Bindematrix nach einem der Ansprüche 1 bis 23 mit mindestens einer Reaktionslösung inkubiert, worin Moleküle von weiteren, an die Bindematrix bindefähigen Reaktionspartnern enthalten sind.

37. Verwendung einer kompartinentierten Bindematrix nach einem der Ansprüche 20 bis 23 oder eines Analysenelements nach einem der Ansprüche 26 oder 27 zum multiplen Analysenachweis.

38. Verwendung einer Bindematrix nach einem der Ansprüche 1 bis 23 oder eines Analysenelements nach einem der Ansprüche 24 bis 27 für Immunoassays.

39. Verwendung einer Bindematrix nach einem der Ansprüche 1 bis 23 oder eines Analysenelements nach einem der Ansprüche 24 bis 27 für die DNA-Diagnostik.

## Claims

1. Binding matrix containing a carrier material with an oxidic surface and a solid phase reactant which is covalently bound thereto by means of anchor groups and which is capable of binding to at least one free reaction partner,
**wherein**
the solid phase reactant forms a diluted and essentially laterally homogenous binding layer on the surface of the carrier material, and the anchor groups are silane groups and are linked to the solid phase reactant via a spacer molecule, and in addition to the spacer molecules linked to the solid phase reactant the binding matrix also contains diluent molecules that are covalently bound to the solid phase by means of anchor groups.

2. Binding matrix as claimed in claim 1,
**wherein**
the coverage of the solid phase reactant on the surface of the carrier material is 0.1 to 90 % of the maximum coverage.

3. Binding matrix as claimed in claim 2,
**wherein**
the coverage of the solid phase reactant on the surface of the carrier material is 0.5 to 70 % of the maximum coverage.

4. Binding matrix as claimed in claim 3,
**wherein**
the coverage of the solid phase reactant on the surface of the carrier material is 1 to 40 % of the maximum coverage.

5. Binding matrix as claimed in one of the claims 1 to 4,
**wherein**
the anchor groups are formed by reaction of the oxidic carrier material surface with a surface reactive silane group of the general formula (I) and (II):
R¹R²R³Si (I)
or 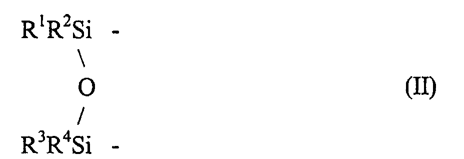 in which R¹, R², R³, R⁴ can be the same or different and denote substituents on the Si atom, provided that at least one of the substituents R¹, R² and R³ in formula (I) or at least one of the substituents R¹ and R² or R³ and R⁴ in formula (II) can covalently bind to an oxidic surface.

6. Binding matrix as claimed in claim 5,
**wherein**
the substituents R¹, R², R³, R⁴ in the silane groups of formulae (I) and (II) are C₁-C₄ alkyl, C₁-C₄ alkoxy, hydroxy, amino group or/and halogen atoms.

7. Binding matrix as claimed in one of the claims 5 or 6,
**wherein,**
all substituents R¹, R², R³, R⁴ can covalently bind to the oxidic surface.

8. Binding matrix as claimed in one of the claims 1 to 7,
**wherein**
the anchor group is linked to the solid phase reactant via a flexible, linear spacer molecule which has a chain length of 2 to 50 atoms.

9. Binding matrix as claimed in claim 8,
**wherein**
the flexible spacer molecule contains an optionally substituted alkylene group or/and an alkylene group which contains heteroatoms.

10. Binding matrix as claimed in one of the claims 1 to 9,
**wherein**
a hydrophilic linker group is located between the spacer molecule and the solid phase reactant.

11. Binding matrix as claimed in claim 10,
**wherein**
the hydrophilic linker group contains one or several oxyethylene groups.

12. Binding matrix as claimed in claim 11,
**wherein**
the hydrophilic linker group is formed by an amino-terminated or hydroxyl-terminated polyethylene oxide.

13. Binding matrix as claimed in claim 12,
**wherein**
the hydrophilic linker group is formed from 1,8-diamino-3,6-dioxaoctane.

14. Binding matrix as claimed in one of the claims 1 to 13,
**wherein**
the diluent molecules have an alkoxy(oligoalkylene oxide) terminal group.

15. Binding matrix as claimed in one of the previous claims,
**wherein**
the solid phase reactant is an antigen or hapten capable of binding to an antibody.

16. Binding matrix as claimed in one of the claims 1 to 14,
**wherein**
the solid phase reactant is biotin or a biotin derivative.

17. Binding matrix as claimed in one of the claims 1 to 14,
**wherein**
the solid phase reactant is composed of an inner and outer component, the outer component being capable of binding to at least one free reaction partner.

18. Binding matrix as claimed in claim 17,
**wherein**
the inner component of the solid phase reactant forms an undiluted layer on the surface of the carrier material and the outer component is coupled to the inner component by affinity binding.

19. Binding matrix as claimed in claim 18,
**wherein**
the inner component is biotin and the outer component is streptavidin.

20. Compartmented binding matrix,
**wherein**
a plurality of spatially separated zones, each of which comprises a binding matrix as claimed in one of the claims 1 to 19 is arranged on a carrier material.

21. Compartmented binding matrix as claimed in claim 20,
**wherein**
the spatially separated zones, each of which comprises a binding matrix, are arranged on a carrier material with a non-oxidic surface.

22. Compartmented binding matrix as claimed in claim 20 or 21,
**wherein**
the spatially separated zones comprise binding matrices each with the same solid phase reactant.

23. Compartmented binding matrix as claimed in claim 20 or 21,
**wherein**
the spatially separated zones comprise binding matrices with different solid phase reactants.

24. Analytical element comprising a diluted binding matrix as claimed in one of the claims 1 to 23,
**wherein**
the solid phase reactant is covered with one or several additional layers of reaction partners.

25. Analytical element as claimed in claim 24,
**wherein**
the reaction partners are selected from antigens, haptens, antibodies or nucleic acids.

26. Analytical element as claimed in claim 24 or 25 for the determination of different analytes in a sample comprising a compartmented binding matrix according to claim 20, wherein the individual spatially separated binding matrix zones are each covered with different further reaction partners.

27. Analytical element as claimed in claim 24 or 25 for the determination of the same analytes in different samples comprising a compartmented binding matrix according to claim 20, wherein the individual spatially separated binding matrix zones are each covered with the same reaction partners.

28. Method for the determination of an analyte in a sample solution by means of a specific binding reaction between at least two reactants with bioaffinity, one of which is present coupled to a solid phase and the other reaction partner or partners are free,
**wherein**
a reactant coupled to the solid phase is used, said reactant being a component of a binding matrix as claimed in one of the claims 1 to 23 or of an analytical elements as claimed in one of the claims 24 to 27.

29. Method as claimed in claim 28,
**wherein**
the specific binding reaction is determined by waveguide measurements or by white-light interferometry.

30. Method as claimed in claim 28 for the concurrent determination of different analytes in a sample solution,
**wherein**
the sample solution is contacted with a compartmented binding matrix as claimed in claim 23 or with an analytical element as claimed in claim 26 and the presence or/and the amount of the various analytes in the sample solution is determined by a locally resolving measurement on the individual spatially separated binding matrix zones.

31. Method as claimed in claim 28 for the parallel determination of an analyte in different sample solutions,
**wherein**
a sample solution is contacted with individual zones of a compartmented binding matrix as claimed in claim 22 or with an analytical element as claimed in claim 27 and the presence or/and amount of the analyte in each sample solution is determined by a locally resolving measurement on the individual spatially separated binding matrix zones.

32. Method as claimed in claim 30 or 31,
**wherein**
the locally resolving measurement is carried out by means of confocal fluorescence microscopy.

33. Process for the production a binding matrix as claimed in one of the claims 1 to 19,
**wherein**
an oxidic carrier material is incubated with a reaction solution which contains molecules that are composed of surface-active silane terminal groups and a solid phase reactant that is linked thereto via a spacer molecule, said reaction solution additionally containing diluent molecules that contain anchor groups which are covalently bound to the solid phase.

34. Process for the production of a compartmented binding matrix as claimed in one of the claims 20 to 23,
**wherein**
spatially separated zones on a carrier material which have an oxidic surface are incubated with the same or different reaction solutions containing molecules that are composed of surface-active silane terminal groups and identical or different solid phase reactants that are linked thereto via spacer molecules.

35. Process as claimed in claim 34,
**wherein**
the spatially separated zones are mounted on a carrier material with a non-oxidic surface.

36. Process for the production of an analytical element as claimed in one of the claims 24 to 27,
**wherein**
a binding matrix as claimed in one of the claims 1 to 23 is incubated with at least one reaction solution which contains molecules of further reaction partners that are capable of binding to the binding matrix.

37. Use of a compound compartmented binding matrix as claimed in one of the claims 20 to 23 or of an analytical element as claimed in one of the claims 26 or 27 for multiple analyte detection.

38. Use of a binding matrix as claimed in one of the claims 1 to 23 or of an analytical element as claimed in one of the claims 24 to 27 for immunoassays.

39. Use of a binding matrix as claimed in one of the claims 1 to 23 or of an analtical element as claimed in one of the claims 24 to 27 for DNA diagnostics.

## Revendications

1. Matrice de liaison contenant un matériau support présentant une surface oxyde et un réactif en phase solide lié par covalence sur celle-ci via des groupes d'ancrage, qui peut se lier avec au moins un partenaire de réaction libre, **caractérisé en ce que** le réactif en phase solide forme une couche de liaison diluée et essentiellement homogène dans le sens latéral sur la surface du matériau support et **en ce que** les groupes d'ancrage sont des groupes silane et sont liés avec les réactifs en phase solide via une molécule d'espacement et **en ce que** la matrice de liaison contient, outre les molécules d'espacement liées avec les réactifs en phase solide, encore des molécules de dilution, qui sont liées par covalence avec les groupes d'ancrage sur la phase solide.

2. Matrice de liaison selon la revendication 1, **caractérisée en ce que** le recouvrement des réactifs en phase solide sur la surface du matériau support représente 0,1 à 90 % du recouvrement maximal.

3. Matrice de liaison selon la revendication 2, **caractérisée en ce que** le recouvrement des réactifs en phase solide sur la surface du matériau support représente 0,5 à 70 % du recouvrement maximal.

4. Matrice de liaison selon la revendication 3, **caractérisée en ce que** le recouvrement des réactifs en phase solide sur la surface du matériau support représente 1 à 40 % du recouvrement maximal.

5. Matrice de liaison selon l'une quelconque des revendications 1 à 4, **caractérisée en ce que** les groupes d'ancrage sont formés par réaction de la surface oxyde du matériau support avec un groupe silane tensioactif de formule générale (I) et/ou (II) :
R¹R²R³Si (I)
ou 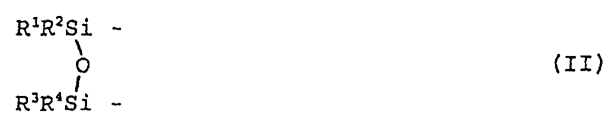 dans laquelle R¹, R², R³, R⁴ sont identiques ou différents et signifient des substituants sur l'atome Si, à condition qu'au moins un des substituants R¹, R² et R³ dans la formule (I) on au moins un des substituants R¹ ou R² ou R³ ou R⁴ dans la formule (II) puissent former une liaison covalente avec une surface oxyde.

6. Matrice de liaison selon la revendication 5, **caractérisée en ce que** les substituants R¹, R², R³, R⁴ dans les groupes silane des formules (I) et (II) sont des groupes alkyle en C₁ à C₄, alcoxy en C₁ à C₄, hydroxy, amino et/ou des atomes d'halogène.

7. Matrice de liaison selon l'une quelconque des revendications 5 ou 6, **caractérisée en ce que** tous les substituants R¹, R², R³, R⁴ peuvent former une liaison covalente avec la surface oxyde.

8. Matrice de liaison selon l'une quelconque des revendications 1 à 7, **caractérisée en ce que** le groupe d'ancrage est lié avec les réactifs en phase solide via une molécule d'espacement souple, linéaire qui présente une longueur de chaîne de 2 à 50 atomes.

9. Matrice de liaison selon la revendication 8, **caractérisée en ce que** la molécule d'espacement souple comprend un groupe alkylène le cas échéant substitué et/ou contenant des hétéroatomes.

10. Matrice de liaison selon l'une quelconque des revendications 1 à 9, **caractérisée en ce qu'**un groupe de liaison hydrophile se trouve entre la molécule d'espacement et le réactif en phase solide.

11. Matrice de liaison selon la revendication 10, **caractérisée en ce que** le groupe de liaison hydrophile contient un ou plusieurs groupes oxyéthylène.

12. Matrice de liaison selon la revendication 11, **caractérisée en ce que** le groupe de liaison hydrophile est formé par un poly(oxyde d'éthylène) terminé par amine ou hydroxyle.

13. Matrice de liaison selon la revendication 12, **caractérisée en ce que** le groupe de liaison hydrophile est formé de 1,8-diamino-3,6-dioxaoctane.

14. Matrice de liaison selon l'une quelconque des revendications 1 à 13, **caractérisée en ce que** les molécules de dilution présentent un groupe terminal alcoxy(oxyde d'oligoalkylène).

15. Matrice de liaison selon l'une quelconque des revendications précédentes, **caractérisée en ce que** le réactif en phase solide est un antigène ou un haptène pouvant se lier à un anticorps.

16. Matrice de liaison selon l'une quelconque des revendications 1 à 14, **caractérisée en ce que** le réactif en phase solide est de la biotine ou un dérivé de la biotine.

17. Matrice de liaison selon l'une quelconque des revendications 1 à 14, **caractérisée en ce que** le réactif en phase solide est constitué d'un composant interne et d'un composant externe, le composant externe pouvant se lier avec au moins un partenaire de réaction libre.

18. Matrice de liaison selon la revendication 17, **caractérisée en ce que** le composant interne du réactif en phase solide forme une couche non diluée sur la surface du matériau support et le composant externe est couplé via une liaison d'affinité au composant interne.

19. Matrice de liaison selon la revendication 18, **caractérisée en ce que** le composant interne est de la biotine et le composant externe de la streptavidine.

20. Matrice de liaison compartimentée, **caractérisée en ce qu'**on a disposé sur un matériau support une multitude de zones séparées dans l'espace qui comprennent à chaque fois une matrice de liaison selon l'une quelconque des revendications 1 à 19.

21. Matrice de liaison compartimentée selon la revendication 20, **caractérisée en ce que** les zones séparées dans l'espace, comprenant à chaque fois une matrice de liaison, sont disposées sur un matériau support présentant une surface non oxyde.

22. Matrice de liaison compartimentée selon la revendication 20 ou 21, **caractérisée en ce que** les zones séparées dans l'espace comprennent des matrices de liaison présentant à chaque fois les mêmes réactifs en phase solide.

23. Matrice de liaison compartimentée selon la revendication 20 ou 21, **caractérisée en ce que** les zones séparées dans l'espace comprennent des matrices de liaison présentant à chaque fois différents réactifs en phase solide.

24. Elément d'analyse, comprenant une matrice de liaison diluée selon l'une quelconque des revendications 1 à 23, **caractérisé en ce que** le réactif en phase solide est recouvert d'une ou de plusieurs couches supplémentaires de partenaires de réaction.

25. Elément d'analyse selon 1a revendication 24, **caractérisé en ce que** les partenaires de réaction sont choisis parmi les antigènes, les haptènes, les anticorps ou les acides nucléiques.

26. Elément d'analyse selon la revendication 24 ou 25 pour la détermination de différents analytes dans un échantillon, comprenant une matricé de liaison compartimentée selon 1a revendication 20, les différentes zones de matrice de liaison séparées dans l'espace étant recouvertes à chaque fois de différents autres partenaires de réaction.

27. Elément d'analyse selon la revendication 24 ou 25 pour la détermination d'analytes identiques dans différents échantillons, comprenant une matrice de liaison compartimentée selon la revendication 20, les différentes zones de matrice de liaison séparées dans l'espace étant recouvertes à chaque fois de partenaires de réaction identiques.

28. Procédé pour la détermination d'un analyte dans une solution d'échantillon au moyen d'une réaction de liaison spécifique entre au moins deux réactifs présentant une bioaffinité, dont l'un se trouve couplé sur une phase solide et le ou les autres partenaires de réaction sont libres, **caractérisé en ce qu'**on utilise un réactif couplé sur la phase solide qui est un constituant d'une matrice de liaison selon l'une quelconque des revendications 1 à 23 ou d'un élément d'analyse selon l'une quelconque des revendications 24 à 27.

29. Procédé selon la revendication 28, **caractérisé en ce qu'**on détermine la réaction de liaison spécifique par des mesures de guidage d'onde ou par interférométrie de la lumière blanche.

30. Procédé selon la revendication 28 pour la détermination en parallèle de différents analytes dans une solution d'échantillon, **caractérisé en ce qu'**on met en contact la solution d'échantillon avec une matrice de liaison compartimentée selon la revendication 23 ou un élément d'analyse selon la revendication 26 et qu'on détermine la présence et/ou la quantité des différents analytes dans la solution d'échantillon par une mesure d'analyse locale sur les différentes zones de matrice de liaison séparées dans l'espace.

31. Procédé selon la revendication 28 pour la détermination en parallèle d'un analyte dans différentes solutions d'échantillon, **caractérisé en ce que** met en contact à chaque fois une solution d'échantillon avec différentes zones d'une matrice de liaison compartimentée selon la revendication 22 ou d'un élément d'analyse selon la revendication 27 et qu'on détermine la présence et/ou la quantité des différents analytes dans chaque solution d'échantillon par une mesure d'analyse locale sur les différentes zones de matrice de liaison séparées dans l'espace.

32. Procédé selon la revendication 30 ou 31, **caractérisé en ce que** la mesure d'analyse locale est réalisée au moyen de microscopie par fluorescence confocale.

33. Procédé pour la fabrication d'une matrice de liaison selon l'une quelconque des revendications 1 à 19, **caractérisé en ce qu'**on incube un matériau support oxyde avec une solution réactionnelle dans laquelle sont contenues des molécules qui sont constituées de groupes terminaux silane tensioactifs et d'un réactif en phase solide lié à ceux-ci via une molécule d'espacement, la solution réactionnelle contenant en outre encore des molécules de dilution qui comprennent des groupes d'ancrage qui sont liés par covalence à la phase solide.

34. Procédé pour la fabrication d'une matrice de liaison compartimentée selon l'une quelconque des revendications 20 à 23, **caractérisé en ce qu'**on incube des zones séparées dans l'espace sur un matériau support, qui présentent une surface oxyde, avec des solutions réactionnelles identiques ou différentes, dans lesquelles sont contenues des molécules qui sont constituées de groupes terminaux silane tensioactifs et de réactifs en phase solide identiques ou différents liés à ceux-ci via des molécules d'espacement.

35. Procédé selon la revendication 34, **caractérisé en ce que** les zones séparées dans l'espace sont appliquées sur un matériau support présentant une surface non oxyde.

36. Procédé pour la fabrication d'un élément d'analyse selon l'une quelconque des revendications 24 à 27, **caractérisé en ce qu'**on incube une matrice de liaison selon l'une quelconque des revendications 1 à 23 avec au moins une solution réactionnelle, dans laquelle sont contenues des molécules d'autres partenaires de réaction pouvant se lier à la matrice de liaison.

37. Utilisation d'une matrice de liaison compartimentée selon l'une quelconque des revendications 20 à 23 ou d'un élément d'analyse selon l'une quelconque des revendications 26 ou 27 pour la mise en évidence multiple par analyse.

38. Utilisation d'une matrice de liaison selon l'une quelconque des revendications 1 à 23 ou d'un élément d'analyse selon l'une quelconque des revendications 24 à 27 pour des immuno-essais.

39. Utilisation d'une matrice de liaison selon l'une quelconque des revendications 1 à 23 ou d'un élément d'analyse selon l'une quelconque des revendications 24 à 27 pour le diagnostic de l'ADN.
